# EUROPEAN PATENT SPECIFICATION

(11) **EP 1 694 183 B1**
(45) Date of publication and mention of the grant of the patent: **16.01.2008**
(21) Application number: 04813833.3
(22) Date of filing: 10.12.2004
(51) Int. Cl.: A47K 7/03

(54) **DISPOSABLE NONWOVEN MITT ADAPTED TO FIT ON A CHILD S HAND**
KINDGERECHTER EINWEGVLIESWASCHHANDSCHUH
MITAINE NON TISSEE JETABLE, CON UE POUR S'ADAPTER A LA MAIN D'UN ENFANT

(30) Priority: 16.12.2003 US 737237
(43) Date of publication of application: 30.08.2006
(73) Proprietor: The Procter and Gamble Company, Cincinnati, Ohio 45202 (US)
(72) Inventor: BENJAMIN, Joyce, Marie, Cincinnati, OH 45240 (US); MASON, Michael, Wayne, Cincinnati, OH 45249-1651 (US); MASON, Jenna, Lebanon, OH 45036 (US)
(74) Representative: Kremer, Véronique Marie Joséphine
(86) International application number: PCT/US2004/041573
(87) International publication number: WO 2005/058119

(56) References cited:
- WO-A-01/08640
- WO-A-01/08641
- WO-A-03/000106
- WO-A-20/04080256
- WO-A-20/04080257
- WO-A-20/04080258
- FR-A- 2 813 777
- US-B1- 6 292 949

## Description

### FIELD OF INVENTION

A disposable nonwoven mitt with a child graphic disposed thereon and releasably carrying a benefit composition and is provided.

### BACKGROUND OF THE INVENTION

Consumer products, such as, and conditioning products as well as household consumer cleaning products, have traditionally been marketed in a variety of forms such as bar soaps, creams, foams, sprays, liquids, powders, lotions, and gels. Typically, these products must satisfy a number of criteria to be acceptable to consumers. These criteria include effectiveness, skin feel, mildness to skin, suitability for use in the consumer's household, and appearance. Typically these consumer products comprise a benefit composition in some form.

It is highly desirable to deliver benefit compositions from a disposable substrate. Disposable products are convenient because they obviate the need to carry or store cumbersome bottles, bars, jars, tubes, and other forms of clutter associated with consumer products. Disposable products are also a more sanitary alternative to the use of a sponge, washcloth, or other implement intended for extensive reuse, because such implements can develop bacterial growth, unpleasant odors, and other undesirable characteristics related to repeated use.

However, while disposable articles, which can be easily used by young children, are desirable they have their own problems. Retention in the hand of a user of such disposable articles, especially during vigorous scrubbing, is one such problem. If a washcloth is prone to fall off during use the user is more concerned with retaining the article in hand instead of actually using the article for its intended purpose.

It can now be appreciated that using consumer products involves many aspects for both the child and the caregiver, especially for the child incapable of reading. Some of these aspects affect children differently, or may not even be a factor for a particular child. It is this uniqueness of each individual child that presents a major challenge for both the child and the caregiver. If any of these aspects are unsuccessful, the child's progress in learning how to bathe or clean properly can be unnecessarily delayed due to numerous failures and frustrations. In the past reusable wash mitts and sponges have been made in various shapes, such as puppets and with child appealing graphics, in order to make the use of these products fun and enjoyable. However, these reusable products still suffer from the problems associated with repeated use, i.e. bacterial growth, unpleasant odors, and other undesirable characteristics related to extensive reuse. One the other hand, while disposable products side step this problem of extensive reuse, no effort has been made to make disposable products more appealing to children.

FR-A-2,813,777 discloses a disposable water proof wash glove containing a graphic and a cleaning composition.

US 6,292,949 describes the preparation of a reusable wash glove constructed from at least two layers of elastic netted material. The glove may contain a graphic.

WO 01/08641 teaches the construction of disposable personal care items comprising a benefit composition. The personal care items are designed to apply the benefit composition to the body.

The problem remains that there is no disposable cleaning articles, products or system available for children of all ages and sizes, which can be easily handled and the method of utilizing easily understood, by the child. The need also remains for disposable cleaning products which are easy to use, appealing to and suitable for use by children, of different ages, all sizes and/or stages of development. Furthermore, the need remains for an article which is retained in a user's hand such that the consumer can focus on the task at hand, namely washing and cleaning, without having to be concerned with retaining the article in their hand.

### SUMMARY OF THE INVENTION

The present invention provides, a disposable nonwoven mitt adapted to fit on a child's hand comprising:
(a) first and second nonwoven sheet members in an overlying relationship, the members defining an interior volume for receiving the child's hand, each of the first and second nonwoven sheet members including an exterior surface, having an opposing interior surface, a top edge, a bottom edge opposing the top edge, and first and second opposed side edges, the first and second nonwoven sheet members being permanently secured to each other along the periphery of the top edge and both of the first and second opposed side edges, with the bottom edges being unsecured so as to provide a substantial access opening to the interior volume for readily inserting the child's hand therein;
(b) a benefit composition, the wherein at least one of the first and second sheet members is releasably carrying the benefit composition; and
(c) child graphic disposed on at least one of the first and second sheet members, and as usage indicator.

All percentages, ratios and proportions are by weight, and all temperatures are in degrees Celsius (°C), unless otherwise specified. All measurements are in SI units unless otherwise specified.

### BRIEF DESCRIPTION OF THE DRAWINGS

The above-mentioned and other features and objects of this invention and the manner of attaining them will become more apparent, and the invention itself will be better understood by reference to the following description of the invention taken in conjunction with the accompanying drawings, wherein:
Figure 1 illustrates one embodiment of a disposable nonwoven mitt adapted to fit on a child's hand.
Figure 2 illustrates a top plan view of the mitt of Figure 1.
Figure 3 is a sectional view along 2-2 of one alternative embodiment of the mitt of Figure 1.
Figure 4 illustrates a bottom plan view of another embodiment of a disposable nonwoven mitt adapted to fit on a child's hand.
Figure 5 illustrates another alternative embodiment of a disposable nonwoven mitt adapted to fit on a child's hand.
Figure 6 illustrates another alternative embodiment of a disposable nonwoven mitt adapted to fit on a child's hand.
Figure 7 illustrates another alternative embodiment of a disposable nonwoven mitt adapted to fit on a child's hand.
Figure 8 illustrates another alternative embodiment of a disposable nonwoven mitt adapted to fit on a child's hand.
Figure 9 illustrates another alternative embodiment of a disposable nonwoven mitt adapted to fit on a child's hand.
Figure 10 illustrates another alternative embodiment of a disposable nonwoven mitt adapted to fit on a child's hand.
Figure 11 illustrates a front view of the mitt of Figure 10.
Figure 12 illustrates one exemplary child graphic.
Figure 13 illustrates an alternative exemplary child graphic..
Figure 14 illustrates an alternative exemplary child graphic.
Figure 15 illustrates one embodiment of a disposable nonwoven mitt adapted to fit on a child's hand having a child graphic thereon.
Figure 16 illustrates an alternative exemplary child graphic.
Figure 17 illustrates an alternative exemplary child graphic.
Figure 18 illustrates an alternative exemplary child graphic.
Figure 19 illustrates an alternative exemplary child graphic.
Figure 20 illustrates an alternative exemplary child graphic.
Figure 21 illustrates an alternative exemplary child graphic.
Figure 22 illustrates an alternative exemplary child graphic.
Figure 23 illustrates an alternative exemplary child graphic.
Figure 24 illustrates an alternative exemplary child graphic.
Figure 25 illustrates an alternative exemplary child graphic.
Figure 26 illustrates an alternative exemplary child graphic.
Figure 27 illustrates an alternative exemplary child graphic.
Figure 28 illustrates an alternative exemplary child graphic.
Figure 29 illustrates an alternative exemplary child graphic.
Figure 30 illustrates an alternative exemplary child graphic.
Figure 31 illustrates an alternative exemplary child graphic.
Figure 32 illustrates an alternative exemplary child graphic.

### DETAILED DESCRIPTION

The instant disposable nonwoven mitt adapted to fit on a child's hand (or disposable nonwoven mitt, nonwoven mitt, or mitt), and methods of the present invention are suitable for use by children. Due to the ease and simple method of use very young children are able to use the instant articles to an extent independently.

### DEFINITIONS

As used herein the abbreviation "gsm" means "grams per square meter".

As used herein, "disposable" is used in its ordinary sense to mean an article that is disposed or discarded after a limited number of usage events, preferably about 2 or less, and more preferably about 1 entire usage events.

The term "releasably carrying" means that a composition is contained in and/or on a nonwoven member and is readily releasable from a nonwoven member by application of water and/or application of some force to the nonwoven member, for example, wringing the disposable child sized article, wiping a child, or immersing part or all of the disposable nonwoven mitt in water.

As used herein, the term "comprising" means that the various components, ingredients, or steps, can be conjointly employed in practicing the present invention. Accordingly, the term "comprising" is open-ended and encompasses the more restrictive terms "consisting essentially of' and "consisting of." Other terms may be defined as they are discussed in greater detail herein.

As used herein, the term "graphic" means any design, shape, pattern, or the like that is or becomes visible on an article, and specifically includes text messages, that include one or more alphanumeric symbol, pictorial images that consist of one or more pictures, and combination thereof.

As used herein, the term "child graphic" means any graphic which appeals to a child such that the child will want to possess and/or interact with the disposable nonwoven mitt in some fashion, such as the typical use to which the disposable nonwoven mitt is put. The child graphic may be aesthetically pleasing, objectively and/or subjectively desirable to any child. The child graphic may in addition be aesthetically pleasing and/or objectively desirable to a child's caregiver. Typically, any child graphic may be supportive and/or encouraging of a child. This support and/or encouragement may be of any suitable subject matter, such as but not limited to, providing advice to the child on any of a range of diverse subjects such as: education e.g. numbers, letters, words, shapes and the like, child appropriate facts and factoids, and combinations thereof; sports and games; jokes, rhymes, limericks humorous stories and the like; social and religious issues, such as but not limited to, sharing and caring, bullying, civics, and the like; safety, such as but not limited to, stranger danger, road safety, hygiene, (i.e. hand washing, bottom wiping and the like); and combinations thereof. One such suitable subject matter of the support and/or encouragement can be with respect to the child's desire to possess and/or interact with the disposable nonwoven mitt in some fashion.

A child graphic can comprise a character or characters. This character may be shown using the disposable nonwoven mitt in an appropriate fashion. The child graphic may additionally include one or more images of the character, characters or parts thereof, performing one or more steps associated with using the disposable nonwoven mitt. Illustrative examples of such step(s) include, but are not limited to: preparation step(s) associated with using the disposable nonwoven mitt, such as but not limited to accessing the mitt and the like; lathering the mitt and the like; using the mitt; and/or disposing of the spent mitt and/or optional container and the like.

Without wishing to be limited to the specific embodiments listed, suitable examples of child graphics may include: a character graphic operating a vehicle, and another child graphic comprising stars, balls, or the like; a character graphic jumping rope, and another child graphic comprising flowers; a character graphic feeding or nurturing and animal, and another child graphic comprising letters of the alphabet; a character graphic holding or using a racquet, bat, glove, other sporting equipment, or illustrated on a sporting field, or the like, and another child graphic comprising objects that are not associated with sports, sporting equipment or the like; a character graphic holding a butterfly net or the like and another child graphic comprising objects that are not associated with butterflies or the like; a character graphic holding a fishing pole, sitting in a boat or the like and another child graphic comprising objects that are not associated with fish, inflatable water toys or the like; a character graphic holding flowers, plants, gardening tools or the like and another child graphic comprising objects that are not associated with flowers, plants or gardening; a character graphic comprising a pet or other animal or an anthropomorphous image feeding, training or nurturing an animal and another child graphic comprising objects that are not associated with pets, animals, animal food, pet toys, or the like; a character graphic playing in a specific environment such as a doll house, barn yard or the like and optionally another child graphic; a character graphic holding or using a telescope or the like and another child graphic comprising objects such as stars, planets or the like; a character graphic comprising a racecar and another child graphic associated with racing; a character graphic comprising a submarine and another child graphic comprising objects associated with fish, bubbles, shells or the like; or other suitable graphics.

The child graphic may vary depending upon the age and/or developmental stage of the child. Typically, this would mean when a graphic is intended for a younger child, typically of approximate age 3 or 4, the graphics will be simpler in nature and comprise bright colors, and typically be easily identifiable and relatable to by a child of that age. The selection of available colors as well as the possible complexity of the child graphics may be increased as the age of the intended child increases. Typically, the older the intended child the more colors, especially subtle colors shades etc, and complex images are available for use on the article.

The child graphic may vary depending upon the gender of the intended child; for example, the child graphic may comprise colors and images which are appealing to girls, such as pinks and images of dolls, rabbits, doll houses and the like or the child graphic may comprise colors and images which are appealing to boys, such as blues and rockets, construction machines, trains and the like. Alternatively, the child graphic may comprise colors and images which are gender neutral and are appealing equally to girls and boys such as purples and greens and cartoon characters, or the child graphic may comprise colors and images which comprise parts which are appealing to boys, parts which are appealing to girls and is overall appealing to both boys and girls.

The term "unrelated in subject matter" is used herein to mean that one graphic is not the same as or is not associated with the subject matter of another graphic. The subject matter relationship or lack thereof can be between two or more text messages, between two or more pictorial images, or between a combination of one or more text messages and one or more pictorial images. The term "text message" means a graphic consisting of one or more alphanumeric symbols, and the term "pictorial image" means a graphic consisting of one or more pictures. The terms "text image" and "pictorial image" are mutually exclusive as used herein.

By way of illustration and without wishing to be limited to the enumerated examples, two pictorial images are considered unrelated in subject matter where the images: illustrate items that are neither identical nor different sizes, shapes, or colors of a common object; illustrate two objects that are not commonly associated with one another, such as an animal and a building block, a jump rope and a flower, a car and a star, a letter of the alphabet and a water toy, a fish and an apple, illustrate items used in unrelated activities, such as items used in sporting activities and items used in gardening activities, or other unrelated activities; or the like. Similarly, two text messages are considered unrelated in subject matter where the messages: are neither identical nor jointly form a sentence, thought, or action; refer to two items that are not commonly associated with one another, such as "ball" and "flower," "fish" and "pencil," "car" and "ghost," or other such unrelated words; or the like. Likewise, a text message and a pictorial image are considered to be unrelated in subject matter where the text does not name, define, describe or otherwise relate to the image.

As used herein, the phrase "related in subject matter" refers to the situation where the subject matter of one graphic is the same as or is associated with the subject matter of another graphic. By way of example, two pictorial images are considered related in subject matter where the images are identical; separately illustrate different sizes, shapes, colors of a common object; each illustrate one and the other of two objects that are commonly associated with one another, such as the moon and stars, a body of water and water toys, a sandbox and suitable toys, a baseball bat and ball, a barn and animals, or the like; illustrate different items used in a particular activity, such as a sporting activity, a gardening activity or the like; jointly illustrate geometrically mating or engaging elements such as a triangle and a triangularly-shaped aperture, or two halves of a zipper; each illustrate one part of a multipart picture; or the like. Similarly, two text messages are considered related in subject matter where the messages: are identical; jointly form a sentence, thought, or action such as "jump" and "up"; each refer to one and the other of two items that are commonly associated with one another, such as but not limited to "bat" and "ball; jointly present a question and answer; or the like. Likewise, a text message and a pictorial image are considered to be related in subject matter where the text names, defines or describes the image; or the like.

The term "disposed on" and variations thereof are intended to mean that one element can be integral with another element, or that one element can be a separate structure bonded to or placed with or placed near another element. Thus, the graphics can be formed or applied directly or indirectly to a surface of a substrate such as but not limited to a nonwoven sheet member, any surface of a container, or other variations or combinations thereof. In particular embodiments, the graphics can be printed, sprayed, or otherwise applied directly on a layer of one of the nonwoven sheet members.

### Disposable Nonwoven Mitt

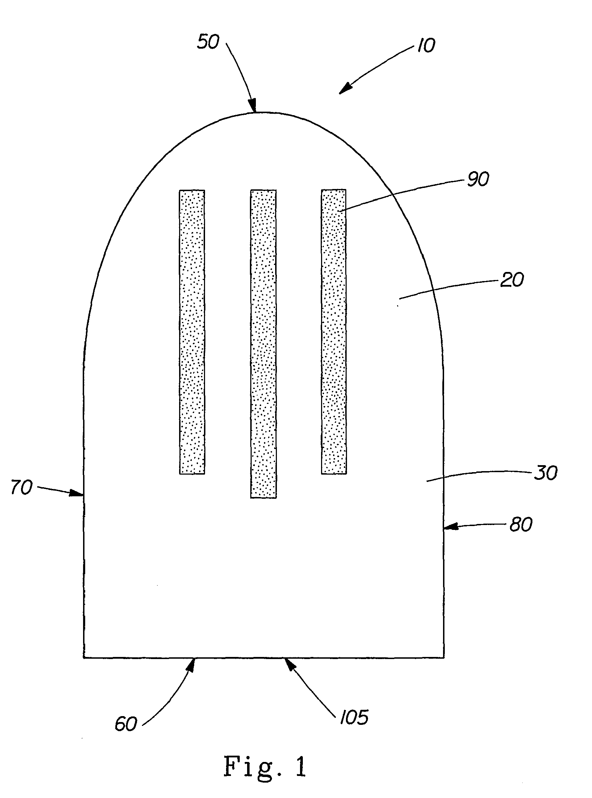

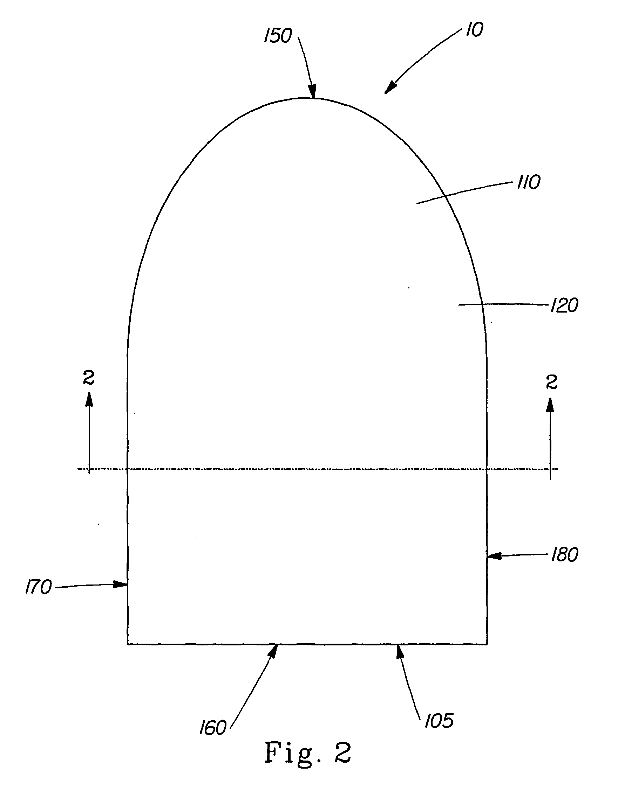

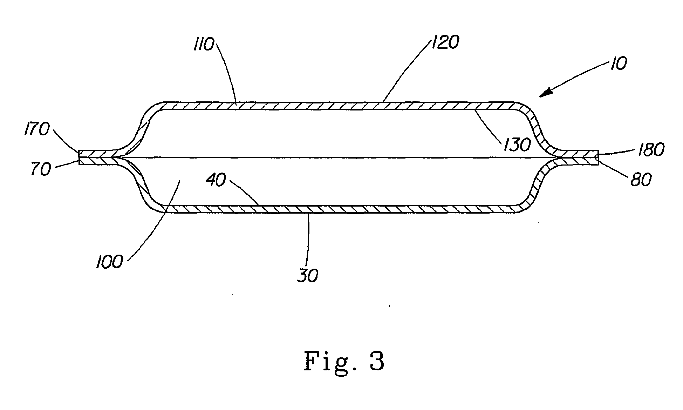

Referring to Figure 1, there is illustrated one possible embodiment of a disposable nonwoven mitt adapted to fit on a child's hand 10, in accordance with the present invention. The disposable nonwoven mitt 10 comprises a first nonwoven sheet member 20 which has an exterior surface 30, an interior surface 40 (Figure 3), a top edge 50, a bottom edge 60, a first side edge 70 and a second side edge 80. The first nonwoven sheet member 20, together with the complementary second nonwoven sheet member 110 (Figure 2), which are in an overlying relationship, define an interior volume 100 (Figure 3) which is accessed by the user's hand via opening 105.

The mitt 10 also comprises a benefit composition 90. The first nonwoven sheet member 20 is releasably carrying a benefit composition 90. In one embodiment of the present invention the benefit composition 90 may be present on a part of the first nonwoven sheet member 20, such as, but not limited to, the exterior surface 30 in the form of stripes, spots, geometric patterns, non-geometric patterns or in a random distribution. In an alternative embodiment, the benefit composition 90 may be present on the entire exterior surface 30 of the first nonwoven sheet member 20. In another an alternative embodiment the benefit composition 90 may be present in the interior of the first nonwoven sheet member, and/or the exterior surface of the nonwoven sheet member.

It is to be understood that while in Figure 1 the exterior surface 30 of the first nonwoven sheet member 20 is releasably carrying the benefit composition 90 in other embodiments of the present invention the second nonwoven sheet member may be releasably carrying the benefit composition. It is to be understood that there is no restriction as to which of the first nonwoven sheet member and the second nonwoven sheet member is releasably carrying the benefit composition. It is even possible that both the first and second nonwoven sheet members be releasably carrying the benefit composition. Furthermore, the benefit composition may be carried on the exterior surface, interior, and/or interior surface of a nonwoven member as long as the nonwoven member is releasably carrying it. The benefit composition, such as but not limited to, type, components, amount and the like, is explained in more detail herein.

Referring to Figure 2, there is illustrated a top plan view of the mitt 10 of Figure 1. The mitt 10 comprises a second nonwoven sheet member 110, which has an exterior surface 120, an interior surface 130 (Figure 3), a top edge 150, a bottom edge 160, a first side edge 170 and a second side edge 180.

Figure 3 is sectional view along 2-2 showing a section view of another alternative embodiment of the mitt of Figure 1. In Figure 3 the first nonwoven sheet member 20 is a single layer material, typically a nonwoven material, such as a high loft batting material which is explained in more detail herein. The first nonwoven sheet member 20 is also releasably carrying the benefit composition which is evenly distributed throughout the first nonwoven sheet member 20.

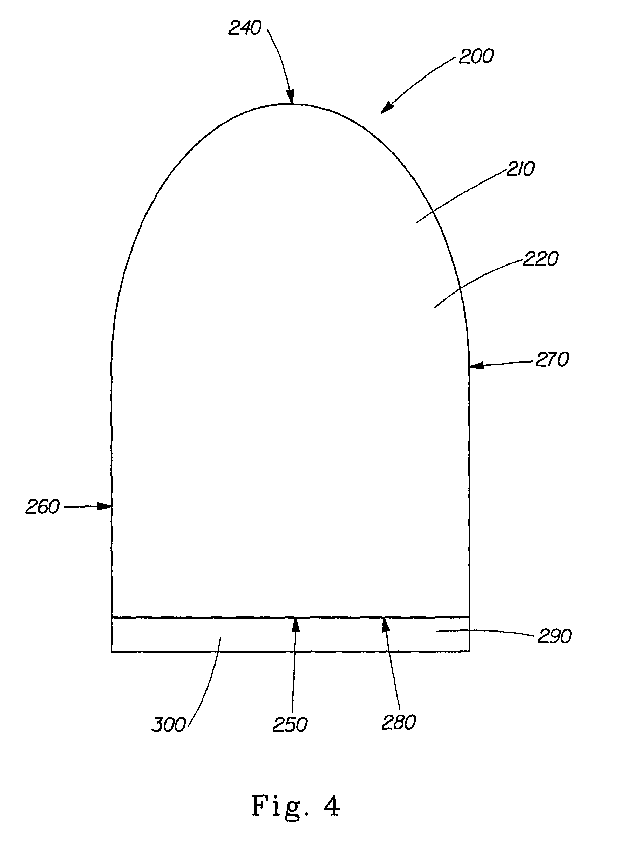

Referring to Figure 4, there is illustrated another one possible embodiment of a mitt 200, in accordance with the present invention. The mitt 200 comprises a first nonwoven sheet member 210, which has an exterior surface 220, an interior surface, a top edge 240, a bottom edge 250, a first side edge 260 and a second side edge 270. The first nonwoven sheet member 210, together with the substantially complementary second nonwoven sheet member 300, which are in an overlying relationship, define an interior volume which is accessed by the hand via 280. However, in this alternative embodiment the second nonwoven sheet member 300 is slightly longer than the first nonwoven sheet member 300 giving rise to an extension portion or tab 290. While not wanting to be limited by theory, this optional tab 290 is believed to provide a visual and tactile clue as to how a user, such as a child, can access the interior volume while also providing assistance in separating the first and second members.

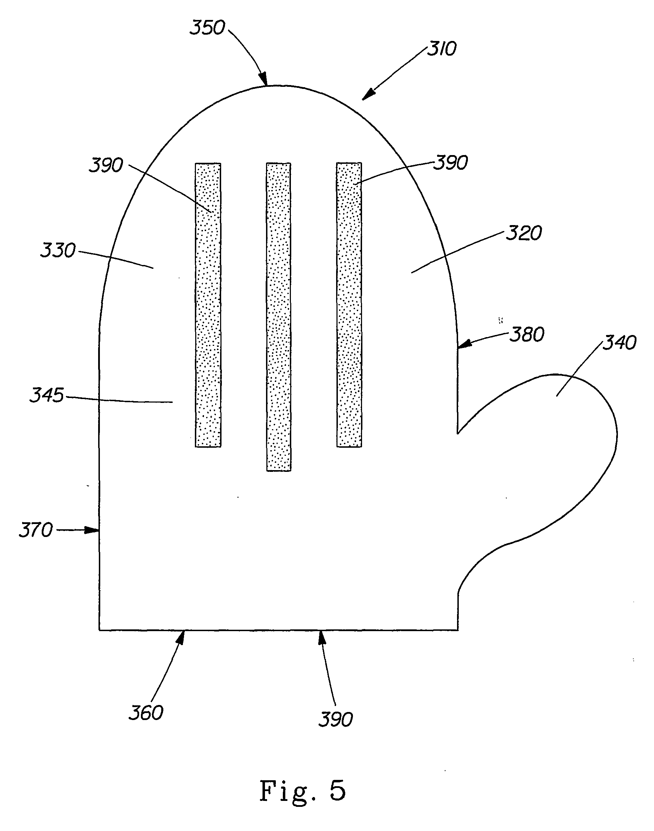

In Figure 5, there is illustrated another possible embodiment of a mitt 310, in accordance with the present invention. The mitt 310 comprises a first nonwoven sheet member 320, which has an exterior surface 330, an interior surface, a top edge 350, a bottom edge 360, a first side edge 370 and a second side edge 380. The first nonwoven sheet member 320, together with a substantially complementary second nonwoven sheet member, which are in an overlying relationship, define an interior volume which is accessed by the user's hand (i.e. a child) via opening 390.

The interior volume of mitt 310 is divided into two parts, one part for the child's thumb 340 and one part for the remainder of the child's hand 345.

The mitt 310 also comprises a benefit composition 390. The first nonwoven sheet member 320 is releasably carrying the benefit composition 390 on its exterior surface 330. In one embodiment of the present invention the benefit composition may be present on a part of the first nonwoven sheet member 320, such as, but not limited to, the exterior surface 330 in the form of stripes, spots, geometric patterns, non-geometric patterns or in a random distribution. In an alternative embodiment, the benefit composition 390 may be present on the entire exterior surface 130 of the first nonwoven sheet member 320. In another an alternative embodiment, not shown, the benefit composition may be present in the interior of the first nonwoven sheet member, and/or the exterior surface of the nonwoven sheet member.

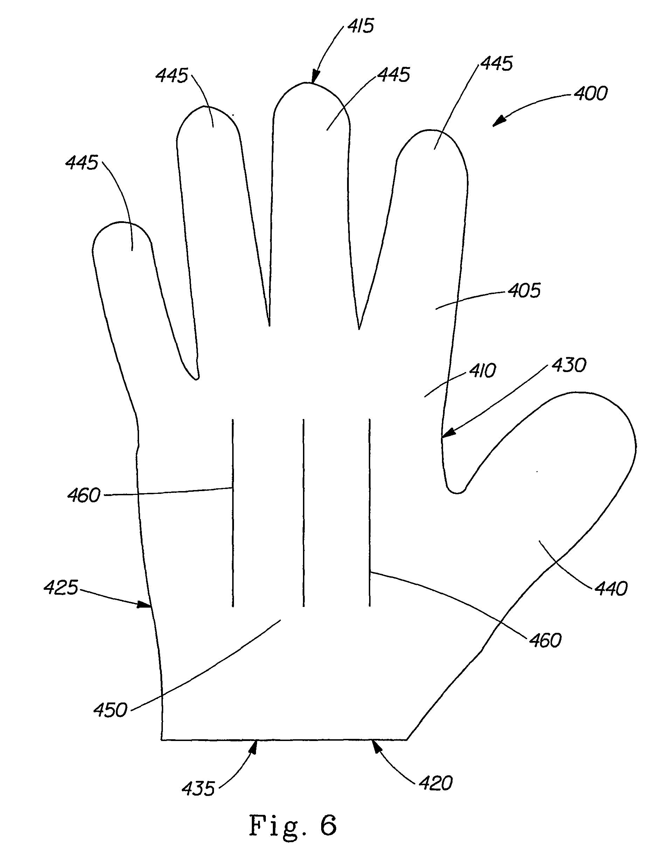

In Figure 6, there is illustrated another possible embodiment of a mitt 400, in accordance with the present invention. The mitt 400 (or glove in this embodiment) comprises a first nonwoven sheet member 405, which has an exterior surface 410, an interior surface, a top edge 415, a bottom edge 420, a first side edge 425 and a second side edge 430. The first nonwoven sheet member 405, together with a substantially complementary second nonwoven sheet member, which are in an overlying relationship, define an interior volume which is accessed by the user's hand (i.e. a child) via opening 435.

The interior volume of mitt 400 is divided into several parts, one part for the child's thumb 440, individual sections for each of the child's fingers 445 and one part for the remainder of the child's hand 450.

The mitt 400 also comprises a benefit composition 460. The first nonwoven sheet member 405 is releasably carrying the benefit composition 460 on its exterior surface 410. In one embodiment of the present invention the benefit composition may be present on a part of the first nonwoven sheet member 405, such as, but not limited to, the exterior surface 410 in the form of stripes, spots, geometric patterns, non-geometric patterns or in a random distribution. In an alternative embodiment, the benefit composition 460 may be present on the entire exterior surface 410 of the first nonwoven sheet member 405. In another an alternative embodiment, not shown, the benefit composition may be present in the interior of the first nonwoven sheet member, and/or the exterior surface of the nonwoven sheet member.

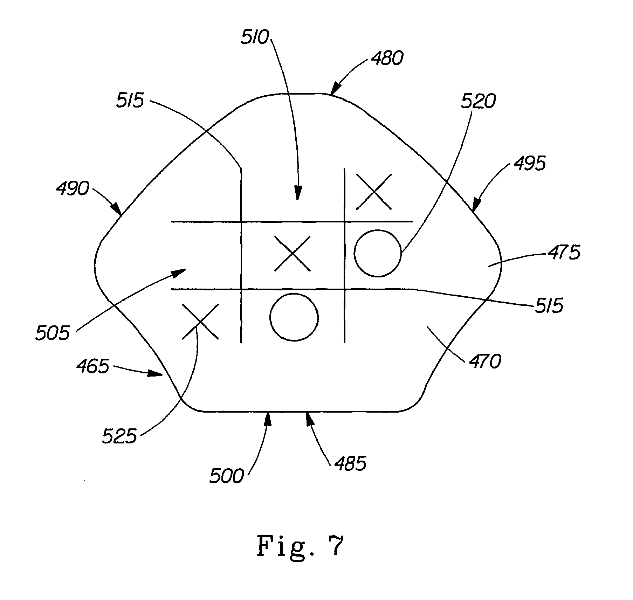

The mitts illustrated in Figures 5 and 6 are "right-handed" mitts. This means that the mitts of Figures 5 and 6 when in use are typically worn on the users (i.e. a child) right hand. However, so-called "left handed" mitts, such as but not limited to, mirror images of the mitts illustrated in Figures 5 and 6, are within the scope of the instant invention and nothing in this disclosure should be in anyway construed as to limit the mitts to either left or right handed. Alternatively, mitts can be "thumb-less", i.e. having a single monolithic or undivided interior volume, thereby allowing the child to place the mitt on either hand. Illustrative non-limiting examples of these types of mitts may be found in Figures 1, 2, 4 and 7. In Figure 7 mitt 465 comprises a first nonwoven sheet member 470, which has an exterior surface 475, an interior surface, a top edge 480, a bottom edge 485, a first side edge 490 and a second side edge 495. The first nonwoven sheet member 470, together with a substantially complementary second nonwoven sheet member, which are in an overlying relationship, define an interior volume which is accessed by the user's hand (i.e. a child) via opening 500.

The mitt 465 also comprises a personal care composition 505, which is present on the exterior surface 475 of the first nonwoven sheet member 470 in the form of a completed game of Tic-Tac-Toe (which is also known as noughts and crosses) 510. The different components of the Tic-Tac-Toe 510, i.e. the lines 515, zeros 520 and x's 525, may be the same color or different, or even comprise different components of the benefit composition 505. In an alternative embodiment, all or some of the zeros 520 and x's 525, may be absent and only the lines 515 which form the grid being present, thereby allowing the child engage in further learning play with the mitt, such as but not limited and imagined game of Tic-Tac-Toe with, for example a care giver, any character graphic present on the mitt, such as one disposed on the second nonwoven member, or any optional mitt container etc. While Tic-Tac-Toe is the only game illustrated in the Figures, it is to be understood that this is to in no way limit the possible arrangement, shapes or images formed on the mitt by the benefit composition. The benefit composition may be arranged on the mitt in any pattern, arrangement, or possible game which may be suitable for a child who would be using the mitt. Non-limiting illustrative examples of possible games, include, Spot the difference in the picture, matching games, crosswords, word puzzles or riddles and the like.

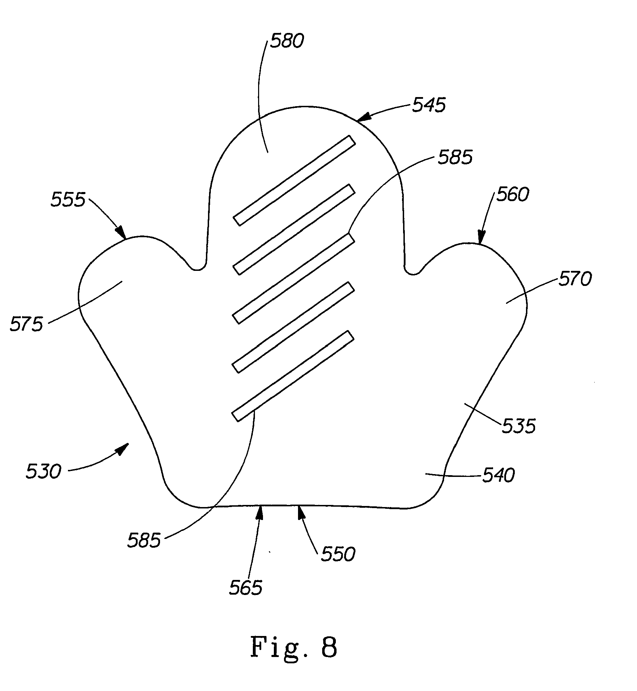

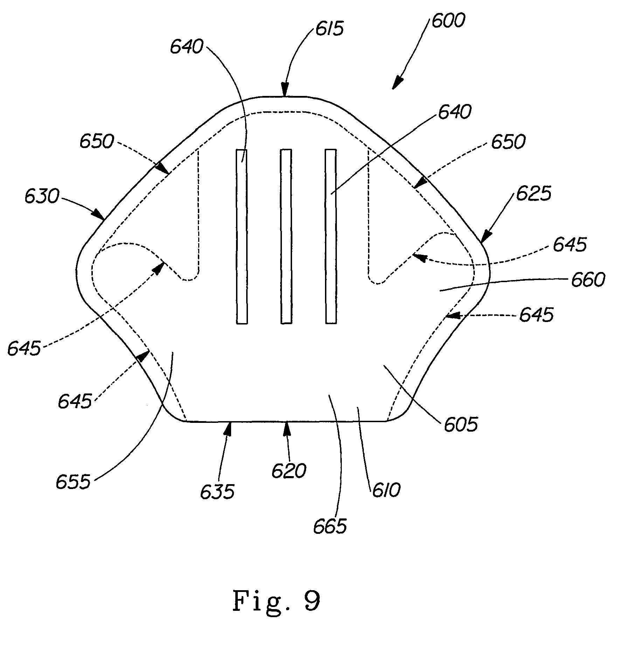

Figure 8 and 9 illustrate mitts which include two opposing thumb portions, thereby enabling the same mitt to be used equally on either the child's right or left hand. In Figure 8 mitt 530 comprises a first nonwoven sheet member 535, which has an exterior surface 540, an interior surface, a top edge 545, a bottom edge 550, a first side edge 555 and a second side edge 560. The first nonwoven sheet member 535, together with a substantially complementary second nonwoven sheet member, which are in an overlying relationship, define an interior volume which is accessed by the user's hand (i.e. a child) via opening 565. The first nonwoven sheet member 535 is releasably carrying a benefit composition 585 on its exterior surface 540.

The interior volume of mitt 530 is divided into several parts, two opposing thumb portions 570 and 575, and one part for the remainder of the child's hand 580.

Figure 9 illustrate a mitt similar to that illustrated in Figure 7. Figure 9 illustrates a mitt 600 which comprises a first nonwoven sheet member 605, which has an exterior surface 610, an interior surface, a top edge 615, a bottom edge 620, a first side edge 625 and a second side edge 630. The first nonwoven sheet member 605, together with a substantially complementary second nonwoven sheet member, which are in an overlying relationship, define an interior volume which is accessed by the user's hand (i.e. a child) via opening 635. The first nonwoven sheet member 605 is releasably carrying a benefit composition 640 on its exterior surface 610. However, unlike the mitt illustrated in Figure 7, the first nonwoven sheet member 605, is joined to the substantially complementary second nonwoven sheet member at join lines 645 and 650 thereby dividing the interior volume of mitt 600 readily accessible by a child's hand in to at least, two opposing thumb portions 655 and 660, and one part for the remainder of the child's hand 665.

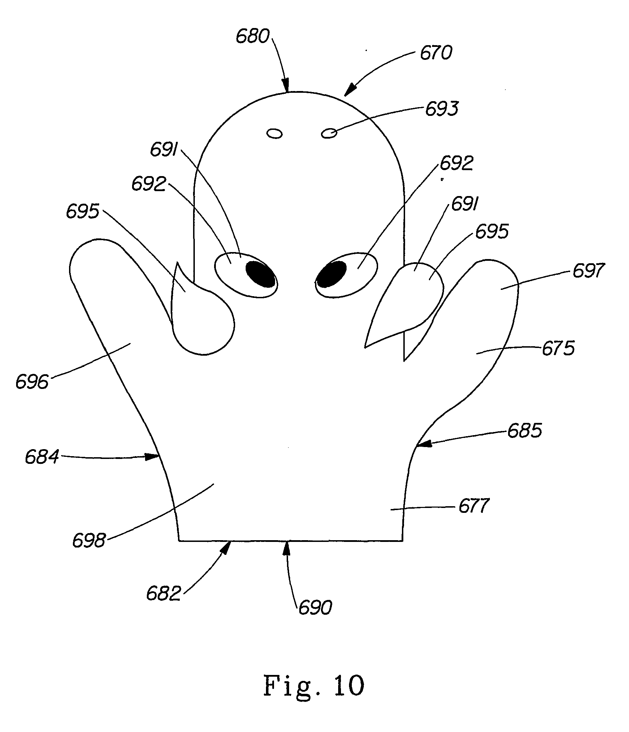

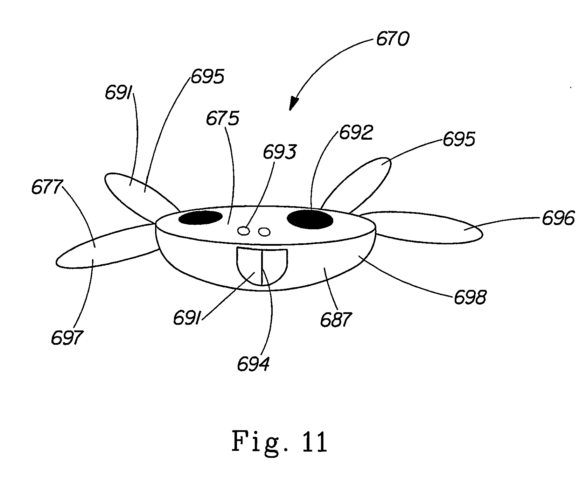

Figures 10 and 11 illustrate another alternative embodiment of the present invention, where the mitt is in the shape of a character or is "puppet-like", in this case in the form of a gnu. The mitt 670 comprises a first nonwoven sheet member 675 which has an exterior surface 677, an interior surface, a top edge 680, a bottom edge 682, a first side edge 684 and a second side edge 685. The first nonwoven sheet member 675, together with a substantially complementary second nonwoven sheet member 687, which are in an overlying relationship, define an interior volume which is accessed by the user's hand (i.e. a child) via opening 690. The first and second nonwoven sheet members 675 and 687 have disposed thereon gnu markings 691, such as eyes 692, nostril 693, teeth 694, ears 695 and the like, to make the mitt 670 appear more "gnuish", i.e. more like a gnu. In one optional embodiment of the present invention part or all of the gnu markings 691 may comprise the benefit composition. In this optional embodiment the gnu markings 691 would at least fade, more preferably disappear over use and provide a caregiver an easy to use system for identifying if the child has been using the mitt 670.

Similarly to the mitt illustrated in Figure 8 the interior volume of mitt 670 is divided into several parts, two opposing thumb portions 696 and 697, and one part for the remainder of the child's hand 698. The two opposing thumb portions 696 and 697 would also include such gnu markings 691 as necessary to create the appearance to a child that the two opposing thumb portions 696 and 697 are the gnu's antlers.

The material of which disposable nonwoven mitt adapted to fit on a child's hand are made from should be strong enough to resist tearing during normal use, yet still provide softness to the child's tender skin. In embodiments that require the mitt to contact water, the material should be water insoluble, or at least capable of retaining its form for the duration of the child's use experience.

The disposable nonwoven mitt may be subjected to various treatments, such as but not limited to, physical treatment, such as zone activation, ring rolling SELFing and the like; chemical treatment, such as rendering part or all of the disposable child sized article hydrophobic, and/or hydrophilic, and the like; thermal treatment, such as softening of fibers by heating, thermal bonding and the like; and combinations thereof.

The disposable nonwoven mitt may be of any size which is suitable for use by a child. Furthermore, the disposable nonwoven mitt may be of a size to be used by a specific developmental age of child, such as 4, or 7and the like. Alternatively, the disposable nonwoven mitt may be of a size which is suitable for use by any child. Typically, the size of the disposable nonwoven mitt will depend upon many factors such as dexterity and hand eye coordination of the child, size of the child's hand, gender, ethnicity, and the like. Anthropomorphic data on child hand sizes may be found in Consumer Safety CHILDATA: Handbook of Child Measurements (Beverly Norris & John Wilson, June 1985). Furthermore, the dimensions and size of the disposable nonwoven mitt will depend upon the shape, weight and composition of the nonwoven sheet members, and the intended use of the mitt. Typically, a thumb less mitt, such as but not limited that illustrated in Figure 1, will have a length of from about 50 mm to about 200 mm, a width of from about 50 mm to about 150 mm a thickness of about 0.01 mm to about 30 mm and The opening to access the interior portion of the mitt may be from about 20 mm to about 200 mm. Alternatively, the area of one side of one of the nonwoven members, such as but not limited to the nonwoven member comprising the benefit composition, has an area of from about 100 mm² to about 30,000 mm².

The disposable nonwoven mitt also comprises a usage indicator. This usage indicator provides a means for the child to readily identify correct usage of the mitt, when all or a portion benefit composition has been released from the nonwoven member(s), and/or they have used the mitt for a sufficient amount of time. The usage indicator may be a separate feature, or it may be part of the benefit composition, or it may be a part of a child graphic, or an entire child graphic when more than one child graphic is present. This type of usage indicator is described further herein. Other suitable usage indicators include, but are not limited to, pH (e.g. at a specific pH or pH range a noticeable event occurs such as color change, noise generation or cessation and the like and combinations thereof), temperature (e.g. the article may feel warm cold for its intended use and then revert to ambient temperature, or change temperature form ambient after a period of time), time (e.g. the indicator may change size shape, color etc after a time period since it was exposed to water air, oxygen, shear or other force and the like), and the like and combinations thereof. In one optional embodiment the usage indicator provides a visual signal during use of the mitt at least a portion of the benefit composition has been released from the mitt and/or nonwoven member.

The type of usage indicator will depend upon many factors, such as but not limited to, size and type of material present in mitt and nonwoven member, benefit composition, intended use of the mitt, age of child using the mitt, the child graphic used and the like. In any event the selection of the usage indicator should not typically not be in isolation from the other elements, such as but not limited to, any character graphic, for example a usage indicator which changes to red, is probably not suitable for younger children because of the possible distress it may possibly cause to a care giver, who thinks the child is possibly hurt, and/or to the child who may think the character is possibly hurt, or a use indictor which changes to green may possibly appear to be gross and slimy to care givers and/or girls (but which may conversely possibly be fascinating and very appealing to boys). In any event selection of usage indicator will depend upon many factors and should not typically be made in isolation of the other components of the mitt.

### Nonwoven Sheet Members

In one embodiment the nonwoven sheet members may be a mixture of natural fibers and synthetic fibers. In alternative embodiments of the present invention the nonwoven sheet members may wholly comprise natural fibers, while in other alternative embodiments still may wholly comprise synthetic fibers.

In one embodiment of the present invention each nonwoven sheet member is made of material which is different to that of the other nonwoven sheet member. In another alternative embodiment of the present invention the two nonwoven sheet members are made of the same material.

Suitable natural fibers include but are not limited to cellulosic fibers, such as wood pulp fibers, cotton, and rayon. Suitable synthetic fibers include fibers commonly used in textiles, including but not limited to polyester and polypropylene fibers polyethylene, polyether, polyethylene terepthalate (PET), and combinations thereof. It is also possible to use bicomponent polymers, or simply bico or sheath polymers. These bico polymers can be used as a component fiber of the nonwoven sheet member, and/or they may be present to act as a binder for the other fibers present in the nonwoven material. Suitable nonwovens with good softness include, but are not limited to, nonwoven materials comprising polypropylene, polyethylene, cellulose, rayon, polyether, PET, bicomponent polymers, and combinations thereof.

Various forming methods can be used to form the nonwoven sheet members for use in the present invention. For instance, the nonwoven sheet members can be made by nonwoven dry forming techniques, such as air-laying, or alternatively by wet laying, such as on a papermaking machine, of a continuous web out of which the nonwoven sheet members are made. Other nonwoven manufacturing techniques, including but not limited to techniques such as adhesive bonding, melt blown, spunbonded, carding, needle punched, hydroentanglement and lamination methods may also be used. Combination of these methods may also be used.

The nonwoven sheet members may be subjected to various treatments, such as, but not limited to, physical treatment, such as zone activation, ring rolling SELFing and the like; chemical treatment, such as, rendering part or all of the nonwoven sheet member hydrophobic, and/or hydrophilic, and the like; thermal treatment, such as softening of fibers by heating, thermal bonding and the like; and combinations thereof.

In one embodiment of the present invention a nonwoven sheet members may comprise a high loft batting material. High loft batting material is a low density, as compared to similar non-high loft nonwoven material, nonwoven comprising a random array of void spaces throughout its structure. High loft batting material is sponge like in its structure and appearance. While not wanting to be limited by theory, it is believed that when a nonwoven sheet member comprises high loft batting material and is releasably carrying the benefit composition there is superior foam generation, such as but not limited to, quality, volume duration etc., compared to a non-high loft batting nonwoven.

In one embodiment one of the nonwoven sheet members is a high loft batting material having a basis weight of from about 50 gsm to about 80 gsm. An example of one suitable nonwoven high loft batting material is a 60 gsm polyester nonwoven, Proef 1297 available from Libeltex of Meulebeke Belgium.

In one embodiment, the nonwoven sheet members may be an airlaid nonwoven material comprising a combination of natural fibers, staple length synthetic fibers and a latex binder. The nonwoven material can be about 20-80 percent by weight wood pulp fibers, 10-60 percent by weight staple length polyester fibers, and about 10-25 percent by weight binder.

In one embodiment of the present invention the surface of a nonwoven sheet member is essentially flat. In another embodiment of the present invention the surface of a nonwoven sheet members may optionally contain raised and/or lowered portions. These can be in the form of logos, indicia, trademarks, geometric patterns, images of the surfaces that the mitt is intended to clean (i.e. infant's body, face, dishes, automobile, etc.,). They may be randomly arranged on the surface of the nonwoven sheet member or be in a repetitive pattern of some form. They may be on one or both of the nonwoven sheet members. In one embodiment one of the nonwoven sheet members contains a repetitive pattern or alternating raised and lowered portions of the substrate. This variation in or on the surface of the nonwoven sheet members may be included to convey to the user, such as a child or a caregiver information on the mitt intended use, how a user, such as a child, is to place the mitt on the their hand, which brand or type of mitt they are using is or even to aid in cleaning of the surface.

It is also within the scope of the present invention that the nonwoven sheet member includes laminates of two or more materials. Commercially available laminates, or purposely built laminates would be within the scope of the present invention. The laminated materials may be joined or bonded together in any suitable fashion, such as but not limited to, ultrasonic bonding, adhesive, glue, fusion bonded, heat or thermal bonded and combinations thereof. One such suitable commercially available laminate is 259-50-3 available from Tredegar of Richmond, Virginia U.S.A.

In one embodiment of the present invention one of the nonwoven sheet members comprises a first material, typically a nonwoven material, which is joined to an elastic web to form a two-layer laminate. In another embodiment of the present invention one of the nonwoven sheet members is a three-layer laminate of two nonwoven materials, forming the outer layers, with an elastic web sandwiched between the nonwoven materials. Additional information on this type of laminate and mitts comprising this type of laminate may be found in copending U.S. Provisional Patent Application No. 60/453,160 filed on March 10, 2003, entitled "Disposable Nonwoven Cleansing Mitt" in the name of Dobrin et al.

In an alternative embodiment of the present invention the one or both of the nonwoven sheet members are selected such that they, preferably their interior surface, will adhere, cling or stick to the child's hand prior to and during use. This optional adhesion may be achieved in a variety of ways, including, but not limited to, adhesive, friction, electrostatic attraction, conformation of the nonwoven sheet member to the shape of the child's hand when wet, and combinations thereof.

In one optional embodiment of the present invention the mitt may include a retaining aid. The retention of the mitts on the user provides the user with additional confidence that the mitt will not fall off during use, especially during vigorous scrubbing. This means the consumer can focus on the task at hand, namely washing and cleaning, without having to be concerned with retaining the mitt on their hand. In one optional embodiment the optional retaining aid is in the form of a polyolefin film attached to the interior surface of one or both of the first and second nonwoven sheet member. Examples of suitable polyolefin films include, but are not limited, films comprising polyethylene and/or polypropylene, and the like. The retaining aid may be discrete swatch or patch, however in alternative embodiments the polymeric film may have substantially the same size and/or shape as the nonwoven member to which it is attached.

In another optional embodiment the retaining aid may be a tacky material, such as an adhesive, may be placed on the interior surface of one or both of the first and second nonwoven sheet members increasing the coefficient of friction between the skin on the user's hand and the disposable nonwoven mitt. This tacky material could be sprayed, printed or the like in the form of dots or other patterns.

Additional information on retaining aids and mitt containing them may be found in copending U.S. Provisional Patent Application No. 60/453,167 filed on March 10, 2003, entitled "Disposable Nonwoven Cleansing Mitt" in the name of Benjamin et al., published as WO 2004/080256 A1.

In another embodiment of the present invention the nonwoven sheet members may biodegradable. For example the substrate could be made from a biodegradable material such as a polyesteramide, or a high wet strength cellulose.

The first and second complementary nonwoven sheet members may be joined or bonded together in any suitable fashion. For example, the first and second nonwoven sheet members may be joined by ultrasonically bonding, sewing, adhesively, mechanically bonding, fusion bonding, heat or thermal bonding and combinations thereof. The first and second nonwoven sheet members are joined at their respective first, second and top edges. The bottom edges may be either totally unbonded or partially bonded. Any such partial bond will not restrict a child from wearing the mitt, and may aid in securing the mitt to the child's hand.

The mitt can be manufactured in various sizes. One embodiment of the present invention include mitts in which the interior volume is divided into two parts, one part for the user's thumb and one part for the remainder of the user's hand. In an alternative embodiment the interior space is not divided and can contain the whole of the user's hand. These embodiment of the present invention are illustrated in the cleansing mitts of the Figures.

In another embodiment of the present invention the disposable mitt is biodegradable. For example the disposable mitt could be made from a biodegradable material, such as a polyesteramide.

Additional information on materials which are suitable for use as the disposable nonwoven mitt, nonwoven sheet members and/or other components thereof can be found in the following patents and applications: U.S. Patent 3,862,472 issued Jan 28, 1975; U.S. Patent 3,982,302 issued Sept. 28, 1976; U.S. Patent 4,004,323 issued Jan. 25, 1977; U.S. Patent 4,057,669 issued Nov. 8, 1977; U.S. Patent 4,097,965 issued July 4, 1978; U.S. Patent 4,176,427 issued Dec. 4, 1979; U.S. Patent 4,130,915 issued Dec. 26, 1978; U.S. Patent 4,135,024 issued Jan. 16, 1979; U.S. Patent 4,189,896 issued Feb. 26, 1980; U.S. Patent 4,207,367 issued June 10, 1980; U.S. Patent 4,296,161 issued Oct. 20, 1981; U.S. Patent 4,309,469 issued Jan 25, 1982; U.S. Patent 4,682,942 issued July 28, 1987; and U.S. Patents 4,637,859; 5,223,096; 5,240,562; 5,556,509; and 5,580,423; and U.S. Patent Application No. US2003/0217425 Published on November 27, 2003 and filed on May 23, 2002 by Datta et al.; and in copending U.S. Provisional Patent Application Nos. 60/453,160 filed on March 10, 2003, entitled "Disposable Nonwoven Cleansing Mitt" in the name of Dobrin et al., and 60/453,167 filed on March 10, 2003, entitled "Disposable Nonwoven Cleansing Mitt" in the name of Benjamin et al., published as WO 2004/ 080256 A1.

The manufacture of disposable nonwoven mitt, nonwoven sheet members and components thereof, such as, nonwoven sheet substrate per se forms no part of this invention.

### Benefit Compositions

The benefit care compositions releasably carried by the disposable nonwoven mitt adapted to fit on a child's hand of the present invention may comprise a variety of components such as are conventionally used in benefit compositions. These benefit compositions and the components thereof should be suitable for application to or use by a child; that is, when incorporated into the disposable nonwoven mitt adapted to fit on a child's hand they are suitable for use in contact with human skin without undue toxicity, incompatibility, irritation, instability, allergic response, and the like, within the scope of sound medical or formulator's judgment.

In one optional embodiment of the present invention the benefit composition may be any composition which is typically used by consumers, such as but not limited to cleaning compositions, moisturizing compositions, medicinal compositions, cosmetic compositions, personal cleansing compositions, germicidal compositions, perfume compositions and combinations thereof.

In one embodiment of the present invention the benefit compositions are in the form of a paste, powder, or a dry solid. While benefit compositions comprising more than about 50% by weight of the composition of a liquid carrier, such as water, are within the scope of the present invention, it is preferred that any mitt be mostly dry, more preferably dry to the touch, prior to contact with the washing environment, that is, until the child first immerses the mitt or otherwise contacts it with water. Typically, this translates into levels of liquid carrier, such as water (not including water of hydration or water similarly bound by the nonwoven sheet material and/or the components of the benefit composition), of less than or equal to about 10%, more preferably less than or equal to about 7% by weight of benefit composition.

In one alternative embodiment of the present invention the amount of benefit composition present in the mitt is preferably present in amounts from about 1 gsm to about 200 gsm, more preferably from about 10 gsm to about 175 gsm, even more preferably still from about 20 gsm to about 150 gsm. (Grams of benefit composition per square meter of nonwoven sheet member) Alternatively, each disposable mitt may preferably contain from about 0.5g to about 20g, more preferably from about 1g to about 15g of benefit composition per disposable nonwoven mitt adapted to fit on a child's hand.

The benefit compositions used in the present invention may contain one or more suitable components. Illustrative, but nonlimiting examples of suitable components of the benefit composition include: surfactant, such as, anionic surfactants, amphoteric surfactants, nonionic surfactants, zwitterionic surfactants, cationic surfactants, and mixtures thereof; enzymes; absorbents; aesthetic components; fragrances; pigments; colorings; colorants; essential oils; skin sensates; anti-acne agents (e.g., resorcinol, sulfur, salicylic acid, erythromycin, zinc, etc.); anti-caking agents; antifoaming agents; preservative; conditioners; hair conditioners; dye; antimicrobial agents (e.g., quaternium-15, paraben preservatives such as but not limited to ethyl paraben, DMDM hydantoin, iodopropyl butylcarbamate(IPBC) etc.); glycerin; binders; buffering agents; bulking agents; chelating agents (e.g., EDTA etc); solvents; cosmetic biocides; denaturants; external analgesics; film formers or materials, e.g., polymers, for aiding the film-forming properties and substantivity of the composition (e.g., copolymer of eicosene and vinyl pyrrolidone); humectants; polydimethylsiloxanes (such as but not limited to dimethicones); Cyclic polyalkylsiloxanes; opacifying agents; pH adjusters; process aids; reducing agents; sequestrants; skin-conditioning agents; moisturizers; skin soothing and/or healing agents (e.g., panthenol and derivatives (e.g., ethyl panthenol); flavonoids (e.g., bioflavonoids, flavones, isoflavones, etc); conditioners (e.g. hair conditioners); hair detanglers; skin treating agents; thickeners (e.g. polymeric thickeners, gums, etc); hydrocolloids; zeolites; sugar amines also known as Amino sugars (e.g. glucosamine, N-acetyl glucosamine, mannosamine, N-acetyl mannosamine, galactosamine, N-acetyl galactosamine, their isomers (e.g., stereoisomers), and their salts (e.g., HCl salt)); phytosterols (e.g. β-sitosterol, campesterol, and the like); oxidants/radical scavengers (such as ascorbic acid (vitamin C), ascorbyl esters of fatty acids, ascorbic acid derivatives (e.g., magnesium ascorbyl phosphate); tea extracts (such as green tea extracts); plant and fruit extracts (e.g. grape skin/seed extracts, melanin, and rosemary extracts, Manjistha, Guggal, kola extract, chamomile, red clover extract, sea whip extract); caffeine; candelilla wax; alpha-bisabolol; aloe vera; allantoin; glycyrrhetic acid; glycyrrhizic acid; abrasives; astringents (e.g., clove oil, menthol, camphor, eucalyptus oil, eugenol, menthyl lactate, witch hazel distillate); gelling agents; thixatropic agents; bulking agents; cosmetic astringents; denaturants; reducing agents; sequestrants; skin bleaching and lightening agents (e.g., hydroquinone); skin treating agents; sunscreen actives (e.g. p-aminobenzoic acid, 2-ethylhexyl-p-methoxycinnamate, 2,2'-dihydroxy-4,4'-dimethoxybenzophenone, zinc oxide, titanium dioxide etc,); and vitamins and derivatives thereof (e.g., tocopherol, tocopherol acetate, beta carotene, nicotinic acid, retinoic acid, retinol, retinoids, retinyl palmitate, niacin, pantothenic, niacinamide, nicotinyl alcohol, and the like); and the like and combinations thereof. The benefit compositions releasably contained by the disposable nonwoven mitt may include carrier components such as are known in the art, for example water, alcohols, polyols, and the like. Such carriers can include one or more compatible liquid or solid filler diluents or vehicles which are suitable for application on to and/or use by a child. Alternatively these carriers may be present in the benefit composition during formulation and application to the nonwoven member and/or disposable nonwoven mitt as a whole and subsequently removed, by any conventional means, such as but not limited to heating, reducing air pressure, and the like.

Some nonlimiting examples of suitable surfactants include ammonium lauroyl sarcosinate, sodium trideceth sulfate, sodium lauroyl sarcosinate, ammonium laureth sulfate, sodium laureth sulfate, ammonium lauryl sulfate, sodium lauryl sulfate, ammonium cocoyl isethionate, sodium cocoyl isethionate, sodium lauroyl isethionate, sodium cetyl sulfate, sodium monolauryl phosphate, sodium cocoglyceryl ether sulfonate, sodium C₉-C₂₂ soap, amine oxides such as lauramine oxide and cocoamine oxide, decyl polyglucose, lauryl polyglucose, sucrose cocoate, C₁₂₋₁₄ glucosamides, sucrose laurate, fatty amines, di-fatty quaternary amines, tri-fatty quaternary amines, imidazolinium quaternary amines, PEG 80 Sorbitan laurate, PEG-150 distearate, sodium laureth-13 carboxylate, disodium lauroamphodiacetate, sodium lauroamphoacetate, cetyl dimethyl betaine, cocoamidopropyl betaine, cocoamidopropyl hydroxy sultaine, and combinations thereof.

Additional information on suitable benefit compositions and possible components thereof may be found in: CTFA Cosmetic Ingredient Handbook, Second Edition (1992); CTFA International Cosmetic Ingredient Dictionary, Fifth Edition, 1993; McCutcheon's, Detergents and Emulsifiers, North American edition (1986), published by Allured Publishing Corporation; McCutcheon's, Functional Materials, North American Edition (1992); Sagarin, et al., Cosmetics Science and Technology (1972); U.S. Patent No. 3,929,678, to Laughlin et al., issued December 30, 1975; U.S. Patent No. 5,833,998 issued to Biedermann et al., on November 10, 1998; U.S. Patent No. 5,939,082 issued to Oblong et al., on Augiust 17, 1999; U.S. Patent 3,755,560, issued August 28, 1973, to Dickert et al.; U.S. Patent 4,421,769, issued December 20, 1983, to Dixon et al.; U.S. Patent No. 4,960,764, to Figueroa, Jr. et al., issued October 2, 1990; U.S. Patent No. 6,335,312 issued on January 1, 2002 to Coffindaffer et al; U.S. Patent No. 5,607,980, issued to McAtee et al, on March 4, 1997; U.S. Patent No. 5,487,884, issued 1/30/96 to Bissett et al.; U.S. Patent No. 5,686,082, issued to N'Guyen on November 1, 1997; U.S. Patent No. 2,831,854, issued to Tucker et al., on April 22, 1958; U.S. Patent No. 4,005,196, to Jandacek, issued January 25, 1977; U.S. Patent No. 4,005,195, to Jandacek, issued January 25, 1977; U.S. Patent No. 5,306,516, to Letton et al, issued April 26, 1994; U.S. Patent No. 5,306,515, to Letton et al, issued April 26, 1994; U.S. Patent No. 5,305,514, to Letton et al, issued April 26, 1994; U.S. Patent No. 4,797,300, to Jandacek et al, issued January 10, 1989; U.S. Patent No. 3,963,699, to Rizzi et al, issued June 15, 1976; U.S. Patent No. 4,518,772, to Volpenhein, issued May 21, 1985; U.S. Patent No. 4,517,360, to Volpenhein, issued May 21, 1985; U.S. Patent No. 4,976,953, to Orr et al, issued December 11, 1990; U.S. Patent No. 5,087,445, to Haffey et al, issued February 11, 1992; U.S. Patent No. 4,509,949, to Huang et al, issued April 5, 1985; U.S. Patent No. 2,798,053, to Brown, issued July 2, 1957; U.S. Patent No. 5,100,660, to Hawe et al, issued March 31, 1992; U.S. Patent No. 4,849,484, to Heard, issued July 18, 1989; U.S. Patent No. 4,835,206, to Farrar et al, issued May 30, 1989; U.S. Patent No. 4,628,078 to Glover et al issued December 9, 1986; U.S. Patent No. 4,599,379 to Flesher et al issued July 8, 1986; U.S. Patent No. 6,200,554, issued to Yeoh et al., on March 13, 2001; U.S. Patent No. 6,248,317 issued to Snyder et al., on June 19, 2001; U.S. Patent No. 4,741,855 issued to Grote et al., on May 3, 1988 and re issued as USRE 34584 on April 12, 1994; U.S. Patent No. 6,280,751 issued to Fletcher et al., on August 28, 2001; U.S. Patent No. 6,506,394 issued to Yohiaoui et al., on January 14, 2003; U.S. Patent No. 6,440,437 issued to Krzysik et al., on August 27, 2002; U.S. Patent No. 6,630,175 issued to Shapiro et al., on October 7, 2003; EP 228,868, to Farrar et al, published July 15, 1987; WO 97/39733 A1, published on October 30, 1997 US. Patent Application No. US20030190337A1: "Methods for regulating the condition of mammalian keratinous tissue via topical application of vitamin B6 compositions", published on October 9, 2003; US. Patent Application No. US20030130636A1: "System for improving skin health of absorbent article wearers", published on July 10, 2003; and US. Patent Application No. US20020177535A1: "Cleansing compositions with milk protein and aromatherapy", published on November 28, 2002.

Mixtures of the above components may also be used.

Additionally, the benefit composition may be applied to a nonwoven sheet member and/or nonwoven mitt in any suitable fashion, such as but not limited to, as a complete benefit composition or at different times in the form of the various components of the benefit composition. Alternatively, the various components of the benefit composition may be placed at different regions on the nonwoven sheet member, e.g. the various gnu markings 342, or tic-tac-toe markings, 510, 515, 520 and 525 may be different components, or mixtures thereof of a benefit composition.

Each of the components of the benefit compositions, when present, are each typically employed in compositions at levels of from about 0.0001% to about 99.9%, preferably from about 0.001% to about 99%, and more preferably from about 0.01% to about 97%, by weight of the benefit composition.

In preparing the mitt the benefit composition needs to be releasably carried by the disposable child sized article, such as placed on and/or in a nonwoven sheet member. Techniques for combining the mitt or nonwoven sheet member with a benefit composition are well known in the art. Examples of common methods of combining a benefit composition with the mitt or nonwoven sheet may involve coating, immersing, dipping, printing, and/or spraying, a nonwoven sheet member with a benefit composition. A benefit composition of is added to the mitt at level sufficient to provide the desired benefits of the present invention. A convenient method of combining a benefit composition with the mitt is for the benefit composition to be applied to a nonwoven sheet member while the nonwoven sheet member is a continuous web. The application could be in many forms, including one or more of, but not limited to coating, immersing, dipping, spraying, printing, extruding and the like. Once the benefit composition is applied the nonwoven sheet member is cut to the desired length and then further processed to form the mitt and then packaged for sale. Alternatively, a benefit composition may be added to a nonwoven sheet member when the nonwoven sheet member is part of a fully or partially formed mitt.

The benefit composition may be added to the mitt in any convenient fashion. For example, the benefit composition components could all be mixed together and then sprayed onto a nonwoven sheet member; each component could be deposited on a nonwoven sheet member separately; or half the components could be mixed together and then added to a nonwoven sheet member, with the remainder then being mixed together and then sprayed on to a nonwoven sheet member.

In one optional embodiment of the present invention the benefit composition is applied to a nonwoven sheet member, in the form of a paste prior to the assembly of the mitt. This optional embodiment is more preferably a "hot melt" composition. Hot melt composition have high viscosity at or around room temperature, and then melt (become substantially liquid) at higher temperatures. Such systems are advantageous during processing of a disposable, substantially dry (or dry to the touch) mitt since the composition can be applied (e.g., coated, sprayed, extruded) to the nonwoven sheet member at a low viscosity (e.g., a liquid) at higher than room temperature, and then as the composition cools down, it becomes a high viscosity paste or solid.

Once the benefit composition is applied to the mitt and/or nonwoven sheet member it may be further treated in any conventional manner, such as but not limited to, heating to remove excess water from the benefit composition.

### Child Graphic

The child graphic may be any suitable visual image or images. The child graphic may include pictorial symbols and/or images, such as but not limited to, photographs, such as but not limited to: a photograph of a child using the disposable nonwoven mitt adapted to fit on a child's hand; drawings, a drawing of a child or an anthropomorphic image of an animal or object using the mitt; cartoons, such as but not limited to, well known cartoon characters, caricatures of famous people, well known brand logos or the like, or characters specifically created to be associated with the article of commerce; symbols, such as but not limited to arrows, indications or motion or movement, and the like; and combinations thereof.

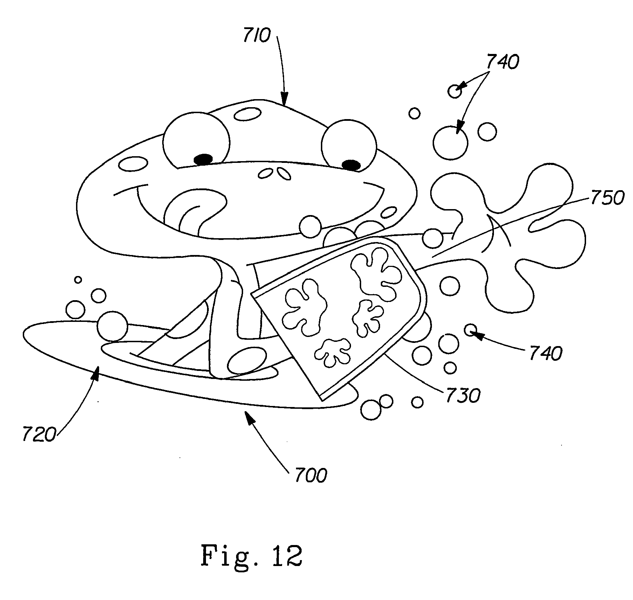

The child graphic may be arranged in any suitable fashion and may be in the form of one or more pictorial images. The arrangement may include the child graphic in, for example, a single image or picture, such as in a single image or a single cartoon. Figure 12 illustrates a single image 700 which includes a child graphic. The child graphics present in image 700 include an anthropomorphic animal 710, in this case an amphibian or frog, who is in a body of water 720, wearing a disposable nonwoven mitt adapted to fit on a child's hand 730 on his hand. It is preferred that the mitt illustrated in the child graphic or child graphics be similar in appearance, at least to a child, to any mitt in association with the child graphic or child graphics. The image further illustrates that frog 720, is cleaning himself thereby generating suds and bubbles 740 using the mitt 730 by contacting parts of his body 750 with the mitt 730.

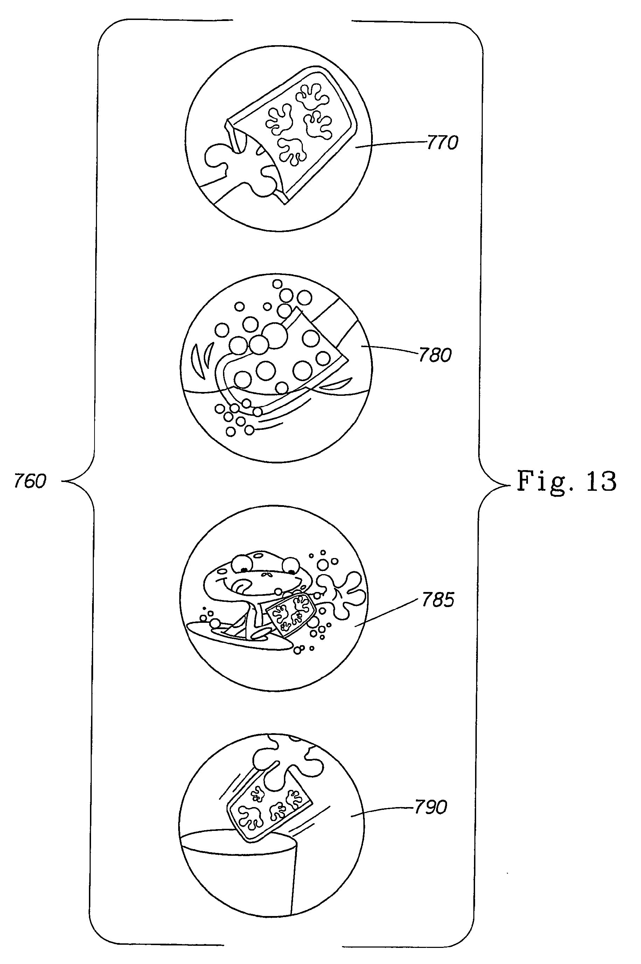

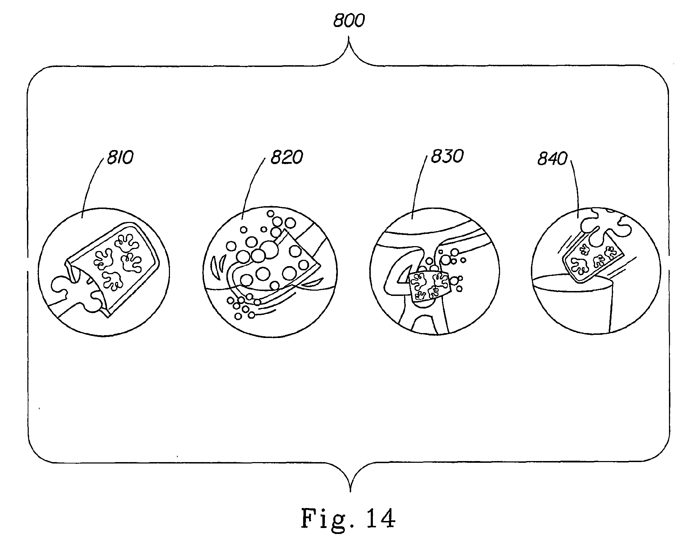

In an optional embodiment of the present invention, the child graphic is a sequential series of panels, wherein each of the panels contains, for example, a different cartoon, symbol, drawing, photograph and combinations thereof. Alternatively, each panel may contain one or more child graphic. These panels may be arranged in any suitable fashion, such as but not limited to, vertically, horizontally, diagonally, circular, and the like and combinations thereof. Examples of this optional embodiment can be found in Figures 13, and 14.

In Figure 13 panels 760 are a child graphic comprising a sequential series of panels communicating to a child incapable of reading how to use a disposable child sized article which is similar to the disposable child sized article illustrated in Figure 1. Panel 770 communicates where the child places its hand and the orientation of the mitt relative to the child. Panel 780 communicates that the child needs to contact the mitt with water, such as by immersion in a body of water such as a bath, or a bucket. Panel 780 also communicates to the child that the benefit composition is only present on one side of the mitt. Additionally, panel 780 reinforces the prior communication in panel 770 on the correct orientation of the mitt relative to the child. Panel 785, that is the frog character, additionally communicates to the child that in order to clean themselves they need to contact, such as by rubbing, scrubbing and the like, their body with the side of the mitt which will generate lather. Additionally, panel 785 further reinforces the prior communication in panels 770 and 780 on the correct orientation of the mitt relative to the child. Panel 790 communicates the need for the child to properly dispose of the mitt after they have finished bathing.

In Figure 14 panels 800 are a sequential series of panels each comprising a different child graphic that are communicating, especially communicating to a child incapable of reading, how to use a mitt which is similar to the mitt illustrated in Figure 4. Panel 810 communicates where the child places its hand to wear the mitt and the correct orientation of the mitt relative to the child. Panel 820 communicates not only that the child needs to contact the mitt with water, such as by immersion in a body of water such as a bath, but that the benefit composition will generate lather when combined with water. Panel 820 also communicates to the child that the benefit composition is only present on one side of the mitt. Additionally, panel 820 reinforces the prior communication in panel 810 on the correct orientation of the mitt relative to the child. Panel 830 reinforces the information communicated in previous panels 810 and 820 by again communicating that the benefit composition will generate lather and is only present on one side of the mitt. Panel 830 additionally communicates to the child that in order to clean themselves they need to contact, such as by rubbing, scrubbing and the like, their body with the side of the mitt which will generate lather. The frog character in panel 830 communicates to the child that the mitt is suitable for use while they are in a bath or similar body of water. Panel 840 communicates the need for the child to properly dispose of the mitt after they have finished bathing.

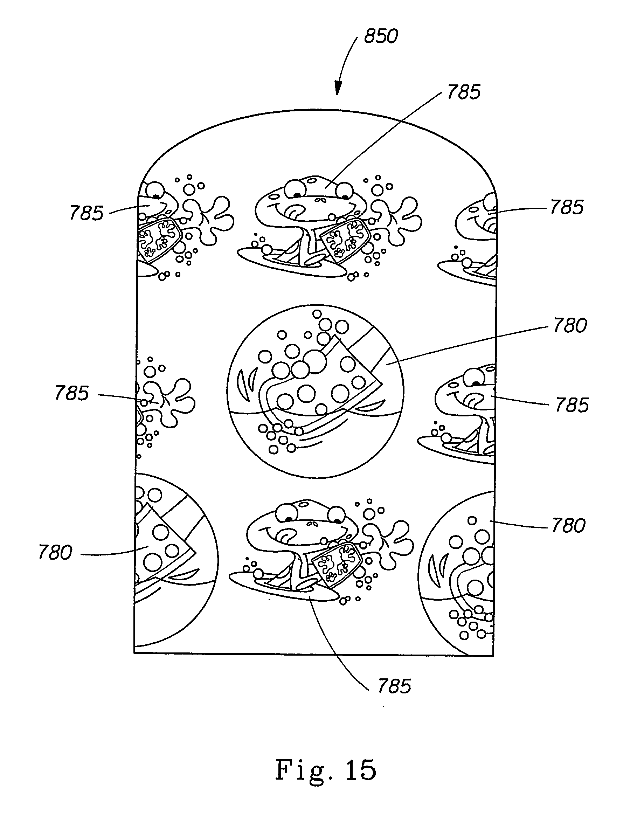

Figure 15 shows a mitt 850 containing a repeating pattern two of the child graphics of Figure 13, namely a repeating pattern of panels 780 and 785. In one optional embodiment mitt 850 may be used in combination with a container which has, for example, printed thereon the entire set, or only panels 770 and 790, of Figure 13. These panels on the mitt 850 would provide additional reinforcement to the child as to the correct use of the mitt 850.

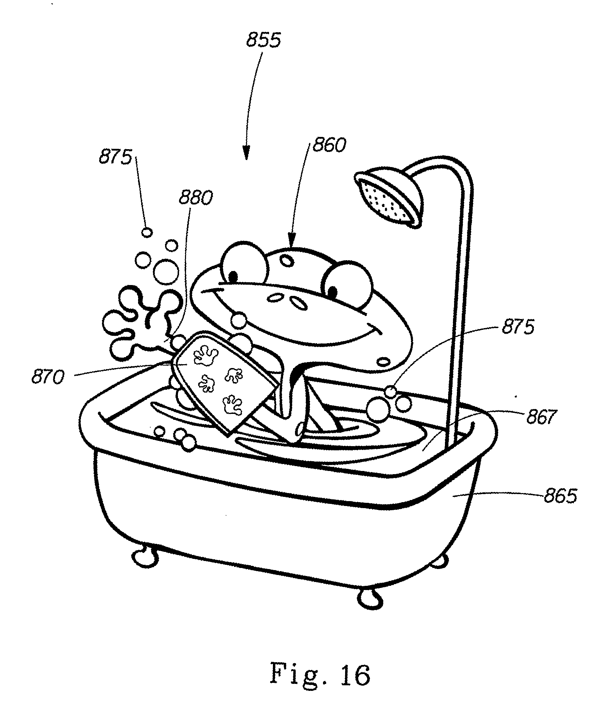

Figure 16 illustrates a single image 855 which includes a child graphic. The child graphics present in image 855 include an anthropomorphic animal 860, in this case a frog, who is in a bath 865 which is full of water 867. The frog 860 is holding a mitt 870 in his hand, which has printed on it a plurality, of proportionally smaller versions of image 855. The image 855 further illustrates that frog 860, is cleaning himself thereby generating suds and bubbles 875 using the mitt 870 by contacting parts of his body 880 with the mitt 870.

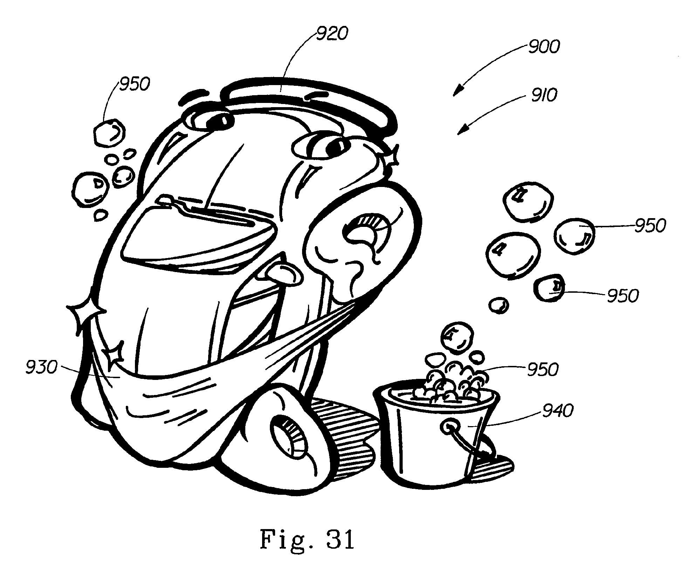

Figure 31 illustrates an image 900 which includes a child graphic 910. The child graphic 910 present in image 900 includes a character graphic, specifically an anthropomorphic object 920, in this case an automobile, who is standing and drying itself with a towel 930. The image 900 further includes a bucket 940 which is full of a liquid which is generating bubbles 950.

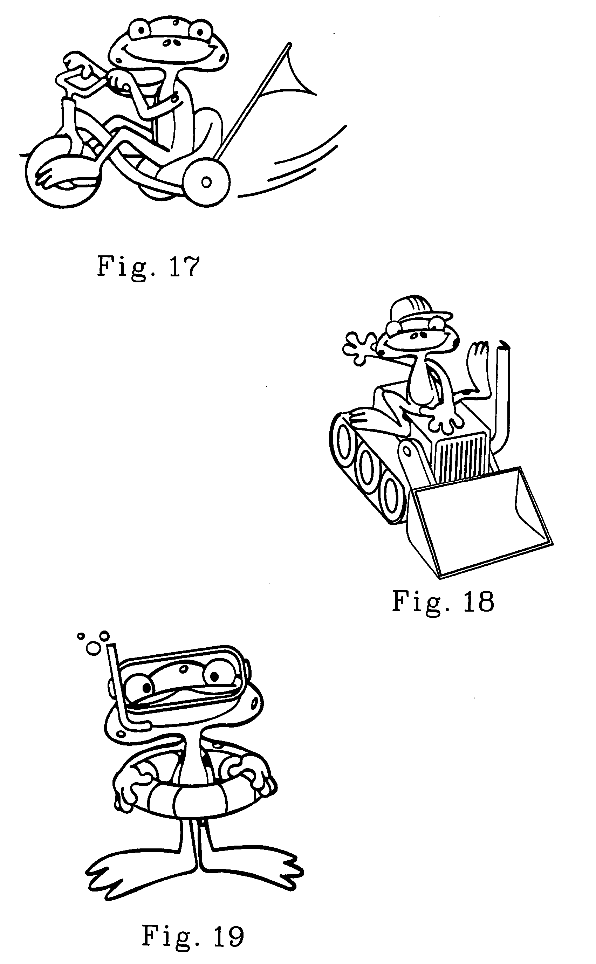

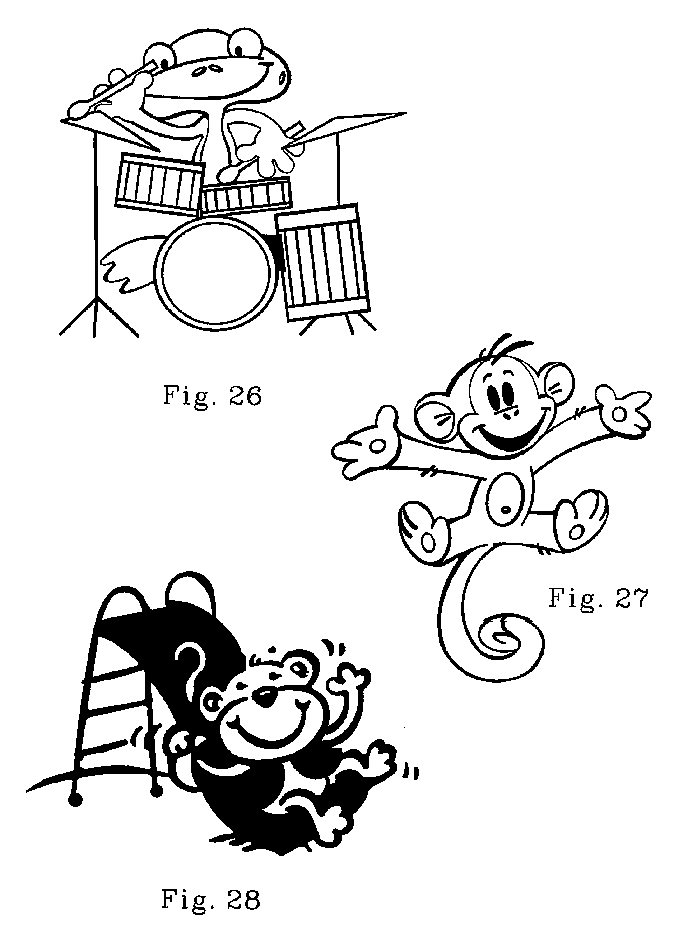

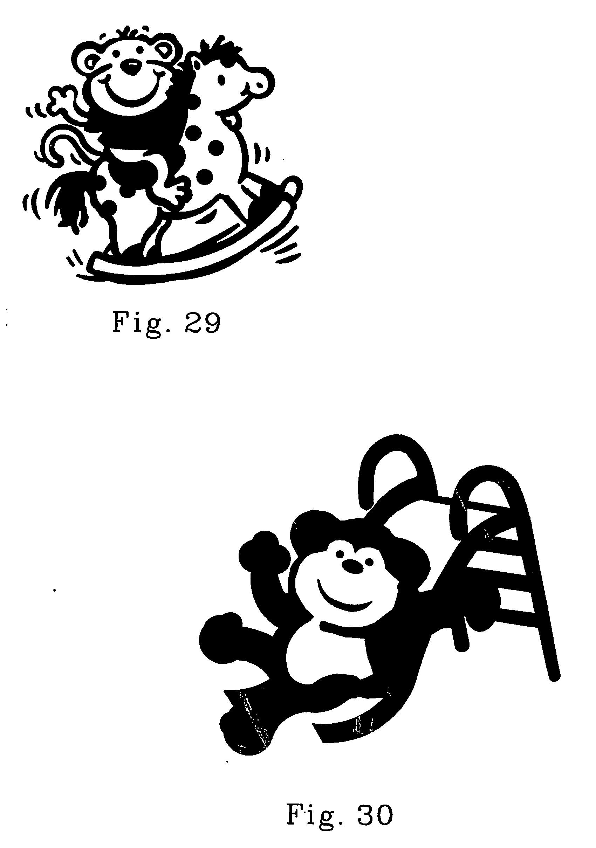

Figures 17 to 32 illustrate additional exemplary child graphics, depicting a character, in these figures frogs, monkeys, turtle or automobile in a range of various activities that a child may typically be engaged in or would readily be able to imaging themselves in that action or activity, either in place of the character or doing the activity along with the character.

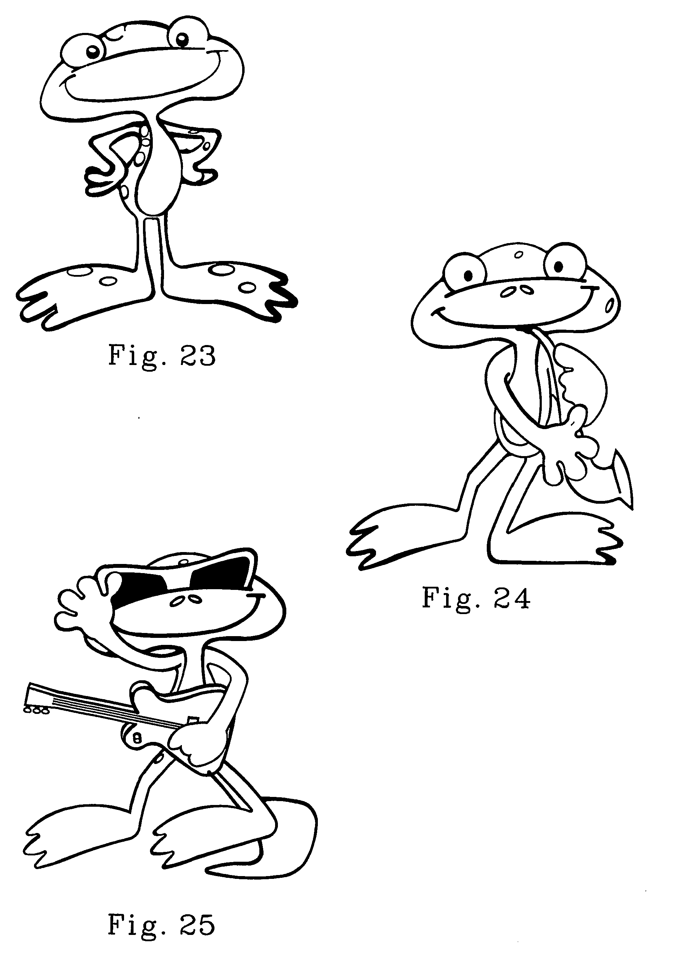

The child graphic or child graphics may also include a story line in which a character, such as the frogs, monkeys, turtle and automobile of Figures 12 to 32, are illustrated performing an activity which may lead to the character needing to perform an activity which may involve the use of the mitt. Illustrative, but non limiting examples of such activities include, running, riding (for example riding a tricycle as illustrated in figure 17 or a bulldozer as illustrated in figure 18), playing in the mud, playing with a ball (figure 23), playing hide and seek, or other similar activities which a child does and can relate to. In this way, the child graphic or child graphics may permit the caregiver to interact with the child regarding the story line created by the child graphic or child graphics and may provide an opportunity for the caregiver to teach the child important life lessons, such as bathing and cleaning, due to the interactive nature of the child graphic.

In one alternative embodiment of the present invention when the mitt comprises two or more child graphics, and/or the mitt is present in a container which has one or more child graphics, these different child graphics may be have a common storyline.

In one alternative embodiment of the present invention when the mitt comprises two or more child graphics, and/or the mitt is present in a container which has one or more child graphics, these different child graphics may be have a related in subject matter.

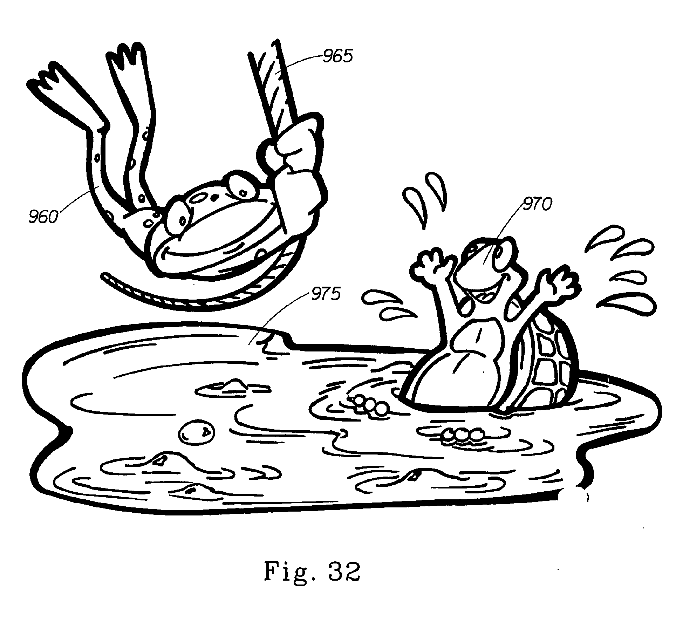

Figure 32 illustrates a pair of child graphics, in this case two character graphics, a frog 960 and a turtle 970 which have a common story line and are related in subject matter. The frog 960 is swinging on a rope 965 over a pool or pond 975 in which the turtle 970 is frolicking and splashing.

In one alternative embodiment when the mitt comprises two or more child graphics, and/or the mitt is present in a container which has one or more child graphics, these different child graphics may be have a unrelated in subject matter.

The child graphic may optionally include a character graphic that can increase the child's interest in using the mitt and can increase the opportunities for the caregiver to interact positively with the child. The term "character graphic" is used herein to refer to a child graphic containing an anthropomorphous image, and in particular an image having or suggesting human form or appearance which ascribes human motivations, characteristics or behavior to inanimate objects, animals, natural phenomena, toys, cartoon characters, or the like. Ideally the character graphic would be suitable for children's swimwear, toys, clothing, diapers or the like and could be utilized to motivate children to use the disposable child sized article. To that end, the character graphics can be associated with popular characters in the media, advertising or well known in a particular culture. Ideally they are characters that the child or caregiver care about and want to identify with. Ideally the child can imagine himself or herself taking the place of the character or emulate the character's behavior/attitude.

The role of the character graphic in the child graphic can be to encourage a child and to motivate them to behaviors, such as but not limited to, cleaning themselves, cleaning their room, and the like. The character graphic may provide a source of entertainment and reassurance for the child and a buddy, or friend, who reduces stress and can be related to in a non-competitive fashion during the training period. The character may also provide positive reinforcement and encouragement to the child while the child is learning new skills and behaviors in a non-competitive or threatening manner.

Suitable character graphics can include animals, people, inanimate objects, natural phenomena, cartoon characters or the like, that can or can not be provided with human features such as arms, legs, facial features or the like. It may be desirable for the character graphic to be familiar to the child, such as an identifiable cartoon character. The character graphics should at least be a type that the child can relate to, examples of which could include animals, toys, licensed characters, or the like. Character graphics can be made more personable and friendly to the child by including human-like features, human-like expressions, apparel, abilities, or the like. In one optional embodiment it is desirable for a character to have a distinguishing feature or features, which in a pictograph can help in training, such as a frog's webbed hand. By way of illustration, an animal character graphic can be shown smiling, wearing clothing, playing sports, fishing, driving, playing with toys, or the like. In particular embodiments, the character graphic can desirably be created to project an appearance that could be described as friendly, positive, non-intimidating, silly, independent, inspirational, active, expressive, dauntless and/or persevering. For example, the frog 710 of Figure 12 is one example of such a character graphic and is intended to inspire the child to learn how to bathe and clean themselves. The frog's expression clearly shows that while he is concentrating on cleaning himself and becoming independent, he is still smiling and having fun. Additionally, it is preferred that the characters expressions are exaggerated so as to not be too subtle for a child to understand.

Furthermore the combination of story line and character graphics are believed to make children more interested in the use of the mitt, such as but not limited to a mitt for use in a bathing or cleaning process, and therefore lead to enhanced results. While not wishing to be limited by theory it is believed that the child graphic and the other elements of the mitt work together to provide to a child, and especially those who are incapable of reading, the appropriate tools, directions in the use of those tools and positive reinforcement which enables the child to learn how to, for example clean themselves, wash their hair, clean their room, and the like.

The character graphic, or parts thereof may retain essentially the same appearance and/or shape while the child is using the disposable nonwoven mitt. Alternatively, the character graphic, or parts thereof may, change appearance, shape, appear and/or disappear while the child is using the disposable nonwoven mitt. That is a use indicator may be optionally a part of a child graphic, or a child graphic when more than one child graphic is present. This change may occur in any suitable manner or fashion, such as but not limited to exposure to a specific environment (e.g. water, air, other suitable chemicals, a pH or pH range), time, abrasion or similar physical force or contact, and the like and combinations thereof. One example of this may be a character who is gesturing hello, welcome or the like, is changed after the child has immersed the mitt in water and uses the mitt, to gesture goodbye.

In one optional embodiment the child graphic may optionally include a character graphic which is associated with a line of children's consumer products, such as but not limited to personal cleansing products and the like. The character may be one of a family, group, team, or the like, each member of which is designed to be associated with, for example, a consumer product, a cleaning event such as washing hair, an age group, stage of infant development and the like. Alternatively, all of the characters of a family, group, team, or the like, may be designed to be associated with the entire range of consumer products.

The association by the child of the character with the consumer product, cleaning event etc., encourages and provides a way for the child to visualize through their imagination the character using the mitt in the way intended. Furthermore, since this teaching is through the use of the child's imagination, there are none of the negative connotations associated with conventional parental instruction on how to use a consumer product, such as the mitt. Instead of the child being subjected to parental nagging to do something the child really doesn't want to do, the child will actively use the mitt as part of active learning play to interact with their new buddy, or friend, and imitate behavior. The interaction between the child and the character is only limited by the bounds of the child's imagination. The role of the caregiver or parent in then becomes one of actively encouraging imaginative play by the child with the character to use the mitt correctly, instead of a being perceived by the child as a parent who stops play. Play is actively encouraged and new skills become part of play, "uninterrupted play". Since the use of the mitt is essentially play, the child is eager to use the mitt and learn the skill.

A family or group of character graphics can be used to progress a child through a system of consumer products, especially systems including the mitt and the like. In this embodiment each character of the family or group, would be tailored to appeal to different groups of children. These groups may be based on age, development stages, regions, etc. Alternatively, a single character may be tailored for one particular group consumer products of line of consumer products which are different for children at different ages, development stages, etc. In this case the character may, for example be, of a different age depending on the consumer product and which group of children the product is intended to be used by.

Child graphics, such as but not limited to character graphics act to enable and encourage the desired behavior, such as the correct use of the disposable child sized article, by providing stimuli. For example, in the case of a child graphic containing a character the stimuli may be entertainment and a friend.

### Container

In one optional embodiment of the present invention the mitt may be present in a container. The container may be any suitable container which is capable of removably holding at least one disposable child sized article. The container may be rigid or it may be semi-rigid. Typically, any container will have a portion for storage of the mitt. The size of the storage portion will depend upon the many factors, such as but not limited to, the size of the disposable child sized article(s), the number of mitt initially present in the container, ease of use by a child, etc. This storage portion may be accessed in any suitable fashion through an opening, or orifice of a size which is suitable for the size of the mitt.

Containers useful include but are not limited, PET tubs, flow wrap pouches, precut sachets for individually packed mitts, and other packaging known in the art as suitable for nonwoven articles releasably carrying a composition, such as but not limited to reach in or so called pop-up containers.

Furthermore, when present, the container may be in the shape of a character, such as, but not limited to a character present in the child graphics present one or more of the mitts.

Additional information on containers, as well as additional option components for containers, including but not limited to: container bodies; lids; containers features, such as but not limited to, attachments of lids, hinges, zippers, securing means; and the like, can be found in U.S. Patent Nos. Des 451,279 issued on December 4,2001, to Chin; Des 437,686 issued on February 20, 2001, to Balzar; Des 443,508 issued on June 12, 2001, to Braaten; Des 443,451 issued on June 12, 2001, to Buck; Des 421,901 issued on March 28, 2000, to Hill; Des 421,902 issued on March 28, 2000, to Hill; Des 416,794 issued on November 23, 1999, to Cormack; Des 414,637 issued on October 5, 1999, to Amundson; Des 445,329 issued on July 24, 2001, to Zethoff; 3,982,659 issued on September 26, 1976, to Ross; 3,967,756 issued on July 6, 1976, to Barish; 3,986,479, issued on October 19, 1976, to Boedecker; 3,994,417 issued on November 30, 1976, to Boedecker; 6,269,970 issued on August 7, 2001, to Huang; 5,785,179 issued on July 28, 1998, to Buczwinski; 5,366,104 issued on November 22, 1994, to Armstrong; 5,322,178 issued on June 21, 1994, to Foos; 5,050,737 issued on September 24, 1991, to Josyln; 4,971,220 issued on November 20, 1990, to Kaufman; 6,296,144 issued on October 2, 2001, to Tanaka; 6,315,114 issued on November 13, 2001, to Keck; 4,840,270 issued on June 20, 1989, to Caputo; 4,471,881 issued on September 18, 1984, to Foster; 5,647,506 issued on July 15, 1997, to Julius; 6,401,968 issued on June 11, 2002, to Huang; 6,269,969 issued on August 7, 2001, to Huang; 6,412,634 issued on July 2, 2002, to Telesca; 5,791,465 issued on August 11, 1998, to Niki; 6,092,690 issued on July 25, 2000, to Bitowft; and 6,092,690 issued on July 25, 2000, to Bitowft; U.S. Patent Application Publication No. 2002/0064323 published on May 30, 2002, inventor Chin; and WO 00/27268 published on May 18, 2000, and assigned to The Procter & Gamble Co.; WO 02/14172 published on February 21, 2002, and assigned to The Procter & Gamble Co.; and WO 99/55213 published on November 4, 1999, and assigned to The Procter & Gamble Co.

### EXAMPLES

Example 1 A cleaning implement which is a mitt and comprising a first member which is a 60 gsm polyester nonwoven high loft batting material, Proef 1297 available from Libeltex of Meulebeke Belgium, and a second member which is a three member laminate. The three member laminate contains a 90 gsm two layer stretch laminate, namely 259-50-3 available from Tredegar, of Richmond, Virginia U.S.A., and a 30 gsm nonwoven, 008YLCO09U, available from BBA of Nashville Tennessee, U.S.A. The high loft batting material is releasably carrying the personal care composition, which is 85 gsm of BC20, available from Rhodia of France. Disposed on the outer surface side of the first nonwoven high loft batting material is the child graphic illustrated in Figure 12.

Example 2 A disposable child sized article according to Example 1, except that the child graphic is that illustrated in Figure 15.

Example 3 A disposable child sized article according to Example 1, except that the child graphic is that illustrated in Figure 16.

Example 4 A cleaning implement which is a mitt and comprising a first member which is a 60 gsm polyester nonwoven high loft batting material, Proef 1297 available from Libeltex of Meulebeke Belgium, around and a second member which is a three member laminate. The three member laminate contains a 90 gsm two layer stretch laminate, namely 259-50-3 available from Tredegar of Richmond, Virginia U.S.A., and a 30 gsm nonwoven, 008YLCO09U, available from BBA of Nashville Tennessee, U.S.A. The high loft batting material is releasably carrying the personal care composition, which is 85 gsm of BC20, available from Rhodia of France. Disposed on the outer surface side of the second nonwoven high loft batting material is the child graphic illustrated in Figure 12.

Example 5 A disposable mitt according to Example 4, except that the child graphic is that illustrated in Figure 14.

Example 6 A disposable mitt according to Example 1, except that personal care composition comprises 15 gsm of a composition as follows:

| **Component** | **%wt.** |
|---|---|
| Sodium Laureth-3 Sulfate | 63.5 |
| Cocamidopropyl Betaine | 23.5 |
| PEG-200 Glyceryl Tallowate | 10.0 |
| Polyquaternium-10 | 1.0 |
| Preservative System | 0.5 |
| Whitener | 0.5 |
| Perfume | 0.5 |
| Water | (Quantity sufficient to 100%) |

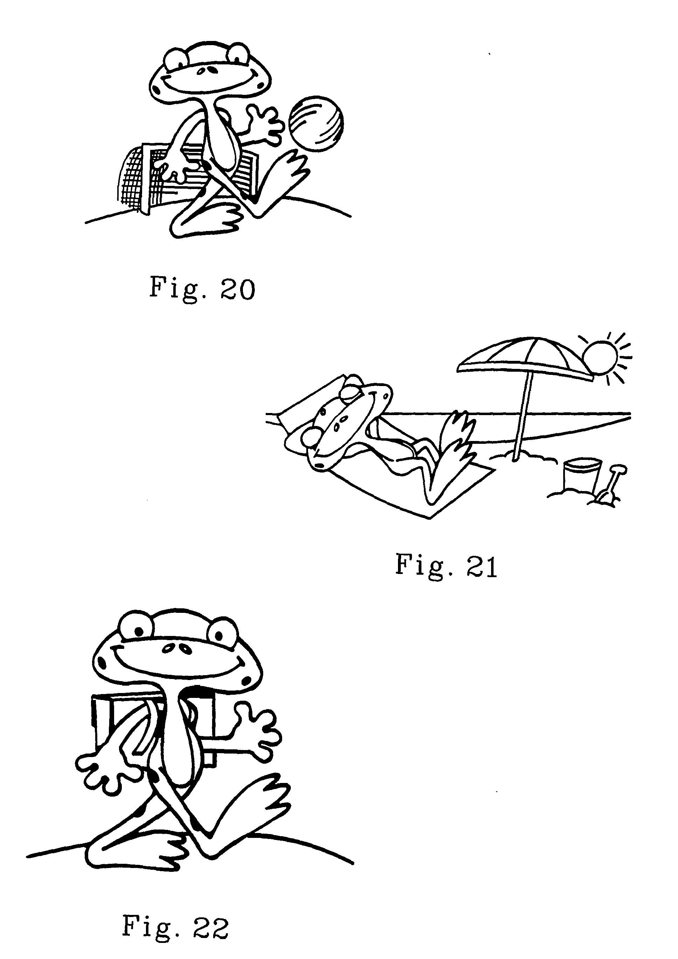

Example 7 A disposable mitt according to example 1 except that the child graphic is that illustrated in Figure 20 and the benefit composition, which is a hair care composition present at a level of 30 gsm, is specifically one of T, U, V or W.

| | T | U | V | W |
|---|---|---|---|---|
| Disodium Lauroamphodiacetate | 0.6 | 0.6 | 0.6 | 0.6 |
| Sodium Trideceth Sulfate | 3.0 | 3.0 | 3.0 | 3.0 |
| PEG-6 Cocamide | -- | 1.0 | -- | -- |
| PEG-6 Lauramide | -- | -- | 1.0 | -- |
| PEG-3 Cocamide | -- | -- | -- | 1.0 |
| Quaternium-22 | 0.37 | 0.37 | 0.37 | 0.37 |
| Glycerin | 1.9 | 1.9 | 1.9 | 1.9 |
| PEG-120 Methyl Glucose Dioleate | 1.0 | 1.0 | 1.0 | 1.0 |
| POE 80 Sorbitan Monolaurate | 3.3 | 3.3 | 3.3 | 3.3 |
| Sodium Laureth-13 Carboxylate | 0.23 | 0.23 | 0.23 | 0.23 |
| Polyquaternium 10 | 0.14 | 0.14 | 0.14 | 0.14 |
| Cocamidopropyl Betaine | 2.8 | 2.8 | 2.8 | 2.8 |
| Tetrasodium EDTA | 0.10 | 0.10 | 0.10 | 0.10 |
| Quaternium 15 | 0.05 | 0.05 | 0.05 | 0.05 |
| Citric Acid, USP | 0.10 | 0.10 | 0.10 | 0.10 |
| Water | --Quantity sufficient | | | |

Example 8 A disposable mitt according to example 4 except that the child graphic is that illustrated in Figure 21 and the benefit composition, which is a sunscreen composition present at a level of 30 gsm, which is prepared in the following fashion: An emulsifier or water-insoluble phase is prepared by blending, at room temperature, cetyl dimethicone copolyol, polyglyceryl-3-distearate, polyglyceryl-4 isostearate, cetyl dimethyl copolyol, hexyl laurate, caprylic and/or capric triglyceride, propylparaben, methylparaben, p-methoxycinnamate, ethylhexyl salicylate, oxybenzone, minor ingredients, and about 0.12% by emulsion weight of a color blend of D&C Green #6, D&C Red #12, and D&C Violet #2 in suitable proportions to make a purple emulsion. Separately, an aqueous phase is prepared by combining, at room temperature, deionized water, aloe vera gel, sodium chloride, chamomile extract, ascorbic acid (Vitamin C), and minor ingredients.

The two phases are then combined by adding the water phase to the emulsifier phase with constant mixing. The mixture is then homogenized with a suitable homogenizing mill until the required viscosity is reached.

The resulting sunscreen is applied to the mitt.

Example 9 A disposable child sized article according to example 1 except that the child graphic is that illustrated in Figure 19 and the benefit composition, which is a hair care composition present at a level of 35 gsm, is specifically one of X, Y, Z, AA or BB.

About one-third to all of the total sulfate surfactant (added as a 25% solution) is added to a jacketed mix tank and heated to about 74°C with slow agitation to form a surfactant solution. Cocamide MEA and fatty alcohol, as applicable, are added to the tank and allowed to disperse. Ethylene glycol distearate (EGDS), as applicable, is then added to the mixing vessel, and melted. After the EGDS is well dispersed (usually about 5 to 20 minutes) polyethylene glycol and the preservative, if used are added and mixed into the surfactant solution. This mixture is passed through a heat exchanger where it is cooled to about 35°C and collected in a finishing tank. As a result of this cooling step, the ethylene glycol distearate crystallizes to form a crystalline network in the product. The remainder of the surfactant and other ingredients including the silicone emulsions are added to the finishing tank with ample agitation to insure a homogeneous mixture. A sufficient amount of the silicone emulsions are added to provide the desired level of dimethicone in the final product. Water dispersible polymers are typically dispersed in water as a 1% to 10% solution before addition to the final mix. Once all ingredients have been added, ammonium xylene sulfonate or additional sodium chloride can be added to the mixture to thin or thicken respectively to achieve a desired product viscosity. Preferred viscosities range from about 2500 to about 9000 cS at 25°C (as measured by a Wells-Brookfield cone and plate viscometer at 15/s). The resulting composition is then applied to the mitt.

| **Component** (% by weight) | **X** | **Y** | **Z** | **AA** | **BB** |
|---|---|---|---|---|---|
| Ammonium AES¹ | 9.00 | 9.00 | 14.0 | 14.85 | 12.50 |
| Cocamidopropylbetaine | 1.70 | 1.70 | 2.70 | 1.85 | 4.20 |
| Polyquaternium-10² | 0.05 | 0.02 | 0.15 | 0.15 | 0.15 |
| Cocamide MEA | 0.80 | 0.80 | 0.80 | 0.80 | 0 |
| Cetyl Alcohol | 0 | 0 | 0.42 | 0.42 | 0.42 |
| Stearyl Alcohol | 0 | 0 | 0.18 | 0.18 | 0.18 |
| Ethylene Glycol Distearate | 1.50 | 1.50 | 1.50 | 1.50 | 1.50 |
| EP Silicone³ | 3.0 | 2.5 | 3.0 | 2.0 | 3.0 |
| Perfume Solution | 0.70 | 0.70 | 0.70 | 0.70 | 0.70 |
| DMDM Hydantoin | 0.37 | 0.37 | 0.37 | 0.37 | 0.37 |
| Color Solution (ppm) | 64 | 64 | 64 | 64 | 64 |
| Water and Minors | --------- q.s. to 100% ---------- | | | | |

| | | | | | |
|---|---|---|---|---|---|
| 1. Ammonium Laureth sulfate having an average ethoxylation of about 3 EO. 2. Polyquaternium-10 is JR30M, a cationic cellulose derived polymer available from Amerchol. 3. EP Silicone is an experimental emulsion polymerized polydimethyl siloxane of about 335,000 csk with particle size of approximately 500 nm made via linear feedstock available from Dow Corning (2-1520; PE106004). | | | | | |

Example 10 A disposable child sized article according to example 1 except that the child graphic is that illustrated in Figure 20 and the benefit composition, which is a sunscreen composition present at a level of 35 gsm, and comprises water, Titanium Dioxide, Xanthan Gum, Cocamide DEA, Neodol 1-7, Neodol 25-7, C12-15 Amine Oxide, glycerin, IPBC, 10,000 cSt. Silicone Fluid, Silicone Antifoam Emulsion, Fragrance2-ethylhexyl-p-methoxycinnamate, 2,2'-dihydroxy-4,4'-dimethoxybenzophenone, zinc oxide, and polyacrtlamide.

## Claims

1. A disposable nonwoven mitt adapted to fit on a child's hand, said mitt comprising:
(a) first and second nonwoven sheet members in an overlying relationship, said members defining an interior volume for receiving said child's hand, each of said first and second nonwoven sheet members including an exterior surface, having an opposing interior surface, a top edge, a bottom edge opposing said top edge, and first and second opposed side edges, said first and second nonwoven sheet members being permanently secured to each other along the periphery of said top edge and both of said first and second opposed side edges, with said bottom edges being unsecured so as to provide a substantial access opening to said interior volume for readily inserting said child's hand therein;
(b) at least one child graphic disposed on at least one of said first and second sheet members, said mitt preferably comprising at least two child graphics;
**characterized in that** said mitt further comprises a benefit composition, wherein at least one of said first and second sheet members releasably carries said benefit composition, said benefit composition preferably being selected from the group consisting of cleaning compositions, moisturizing compositions, medicinal compositions, cosmetic compositions, personal cleansing compositions, germicidal compositions, perfume compositions, and combinations thereof ;
(c) a usage indicator, said usage indicator preferably providing a visual signal during use of said article that at least a portion of said benefit composition has been released from said nonwoven member, said usage indicator more preferably being selected from the group consisting of pH change indicator, a temperature change indicator, time change indicator and combinations thereof.

2. The disposable nonwoven mitt according to Claim 1 wherein said child graphic is disposed on said first nonwoven sheet member.

3. The disposable nonwoven mitt according to Claim 1 wherein said child graphic is disposed on said second nonwoven sheet member.

4. The disposable nonwoven mitt according to any of the above claims wherein said child graphic is in the form of photographs, drawings, cartoons, symbols and combinations thereof.

5. The disposable nonwoven mitt according to Claim 1 wherein said disposable nonwoven mitt is thumb-less.

6. The disposable nonwoven mitt according to any of Claims 1-4 wherein said interior volume is divided into at least two portions wherein at least one of said portions is adapted to surround a child's thumb

7. The disposable nonwoven mitt according to any of the above claims wherein said child graphic is appealing to a child of from about three years old to about five years old.

8. The disposable nonwoven mitt according to any of Claims 1-6 wherein said child graphic is appealing to a child of from about six years old to about nine years old.

9. The disposable nonwoven mitt according to any of the above claims wherein said child graphic is selected from the group consisting of graphics appealing to girls, graphics appealing to boys' gender neutral graphics and graphics appealing to either gender.

10. The disposable nonwoven mitt according to any of the above claims wherein said child graphic comprises a character graphic, said character graphic preferably having a form selected from the group consisting of photographs, drawings, cartoons, symbols and combinations thereof, said character graphic more preferably being in the form of a cartoon selected from the group consisting of cartoons of animals, cartoons of objects and combinations thereof, and most preferably said character graphic is a cartoon of an animal, wherein the animal is selected from the group consisting of amphibians, turtles, monkeys, automobiles and combination thereof.

11. The disposable nonwoven mitt according to any of the above claims wherein said child graphic is a registered graphic.

12. The disposable nonwoven mitt according to any of the above claims wherein at least a portion of least one of said first and second nonwoven sheet members comprises at least one raised region, said raised region preferably having a form selected from the group consisting of logos, indicia, trademarks, geometric patterns, images and combinations thereof.

13. The disposable nonwoven mitt according to Claims 1-11 wherein at least a portion of least one of said first and second nonwoven sheet members comprises at least one lowered region, said lowered region preferably region preferably having a form selected from the group consisting of logos, indicia, trademarks, geometric patterns, images and combinations thereof.

14. The disposable nonwoven mitt according to Claims 12 or 13 wherein said form is arranged randomly on least one of said first and second sheet members.

15. The disposable nonwoven mitt according to Claims 12 or 13 wherein said form is arranged in a repetitive pattern.

16. The disposable nonwoven mitt according to Claims 12 or 13 wherein said child graphic is substantially complementary with one of said at least of said forms.

17. The disposable nonwoven mitt according to Claim 1 wherein said usage indicator is a child graphic.

18. The disposable nonwoven mitt according to any of the above claims wherein said disposable nonwoven mitt is one component of a system of consumer products.

## Patentansprüche

1. Einweg-Fausthandschuh aus Vliesstoff, der so ausgelegt ist, dass er an eine Kinderhand passt, wobei der Fausthandschuh Folgendes umfasst:
(a) aufeinander liegende erste and zweite flächige Vlieselemente, die ein Innenvolumen zum Aufnehmen der Kinderhand bilden, wobei jedes der ersten und zweiten flächigen Vlieselemente eine Außenfläche mit einer entgegengesetzten Innenfläche, einem oberen Rand, einem dem oberen Rand entgegengesetzten unteren Rand und ersten und zweiten entgegengesetzten Seitenrändern aufweist, wobei die ersten und zweiten flächigen Vlieselemente entlang des Umfangs des oberen Rands und der ersten und zweiten entgegengesetzten Seitenränder dauerhaft aneinander befestigt sind, wobei die unteren Ränder nicht befestigt sind, so dass sie eine erhebliche Einführöffnung in das Innenvolumen bieten, so dass die Kinderhand leicht darin eingeführt werden kann;
(b) mindestens eine auf zumindest einer der ersten und zweiten Flächenelemente angeordnete kindgerechte Grafik, wobei der Fausthandschuh vorzugsweise mindestens zwei kindgerechte Grafiken umfasst;
**dadurch gekennzeichnet, dass** der Fausthandschuh ferner eine vorteilhafte Zusammensetzung umfasst, wobei mindestens eines der ersten und zweiten Flächenelemente die vorteilhafte Zusammensetzung lösbar trägt, wobei die vorteilhafte Zusammensetzung vorzugsweise ausgewählt ist aus der Gruppe bestehend aus Reinigungszusammensetzungen, Feuchtigkeitszusammensetzungen, medizinischen Zusammensetzungen, kosmetischen Zusammensetzungen, Körperreinigungszusammensetzungen, keimtötenden Zusammensetzungen, Duftstoffzusammensetzungen und Kombinationen davon ist;
(c) einen Gebrauchsindikator, wobei der Gebrauchsindikator vorzugsweise während des Gebrauchs des Artikels ein optisches Signal liefert, dass mindestens ein Teil der vorteilhaften Zusammensetzung aus dem Vliesstoffelement freigesetzt wurde, wobei der Gebrauchsindikator mehr bevorzugt ausgewählt ist aus der Gruppe bestehend aus pH-Änderungsindikator, Temperaturänderungsindikator, Zeitänderungsindikator und Kombinationen davon.

2. Einweg-Fausthandschuh aus Vliesstoff nach Anspruch 1, wobei die kindgerechte Grafik auf dem ersten Vlieslagenelement angeordnet ist.

3. Einweg-Fausthandschuh aus Vliesstoff nach Anspruch 1, wobei die kindgerechte Grafik auf dem zweiten flächigen Vlieselement angeordnet ist.

4. Einweg-Fausthandschuh aus Vliesstoff nach einem der vorgenannten Ansprüche, wobei die kindgerechte Grafik in Form von Fotoaufnahmen, Zeichnungen, Cartoons, Symbolen und Kombinationen davon vorliegt.

5. Einweg-Fausthandschuh aus Vliesstoff nach Anspruch 1, wobei der Einweg-Fausthandschuh aus Vliesstoff daumenlos ist.

6. Einweg-Fausthandschuh aus Vliesstoff nach einem der Ansprüche 1-4, wobei das Innenvolumen in mindestens zwei Abschnitte unterteilt ist, wobei mindestens einer der Abschnitte so ausgelegt ist, dass er den Daumen eines Kindes umgeben kann.

7. Einweg-Fausthandschuh aus Vliesstoff nach einem der vorgenannten Ansprüche, wobei die kindgerechte Grafik ein Kind von etwa drei bis etwa fünf Jahren anspricht.

8. Einweg-Fausthandschuh aus Vliesstoff nach einem der Ansprüche 1-6, wobei die kindgerechte Grafik ein Kind von etwa sechs bis etwa neun Jahren anspricht.

9. Einweg-Fausthandschuh aus Vliesstoff nach einem der vorgenannten Ansprüche, wobei die kindgerechte Grafik ausgewählt ist aus der Gruppe bestehend aus Grafiken, die Mädchen ansprechen, Grafiken, die Jungen ansprechen, geschlechtsneutralen Grafiken und Grafiken, die beide Geschlechter ansprechen.

10. Einweg-Fausthandschuh aus Vliesstoff nach einem der vorgenannten Ansprüche, wobei die kindgerechte Grafik eine Zeichengrafik umfasst, wobei die Zeichengrafik vorzugsweise eine Form aufweist, die ausgewählt ist aus der Gruppe bestehend aus Fotoaufnahmen, Zeichnungen, Cartoons, Symbolen und Kombinationen davon, wobei die Zeichengrafik mehr bevorzugt in Form eines Cartoons vorliegt, der ausgewählt ist aus der Gruppe bestehend aus Tiercartoons, Objektcartoons und Kombinationen davon und wobei die Zeichengrafik am meisten bevorzugt ein Tiercartoon ist, wobei das Tier ausgewählt ist aus der Gruppe bestehend aus Amphibien, Schildkröten, Affen, Fahrzeugen und Kombinationen davon.

11. Einweg-Fausthandschuh aus Vliesstoff nach einem der vorgenannten Ansprüche, wobei die kindgerechte Grafik eine eingetragene Grafik ist.

12. Einweg-Fausthandschuh aus Vliesstoff nach einem der vorgenannten Ansprüche, wobei mindestens ein Teil von zumindest einem der ersten und zweiten flächigen Vlieselemente mindestens einen erhabenen Bereich umfasst, wobei der erhabene Bereich vorzugsweise eine Form aufweist, die ausgewählt ist aus der Gruppe bestehend aus Logos, Anzeigen, Warenzeichen, geometrischen Mustern, Bildern und Kombinationen davon.

13. Einweg-Fausthandschuh aus Vliesstoff nach den Ansprüchen 1-11, wobei mindestens ein Teil von zumindest einem der ersten und zweiten flächigen Vlieselemente mindestens einen vertieften Bereich umfasst, wobei der vertiefte Bereich vorzugsweise eine Form aufweist, die ausgewählt ist aus der Gruppe bestehend aus Logos, Anzeigen, Warenzeichen, geometrischen Mustern, Bildern und Kombinationen davon.

14. Einweg-Fausthandschuh aus Vliesstoff nach den Ansprüchen 12 oder 13, wobei die Form auf mindestens einem der ersten und zweiten Flächenelemente nach Zufallsprinzip angeordnet ist.

15. Einweg-Fausthandschuh aus Vliesstoff nach den Ansprüchen 12 oder 13, wobei die Form in einem sich wiederholenden Muster angeordnet ist.

16. Einweg-Fausthandschuh aus Vliesstoff nach den Ansprüchen 12 oder 13, wobei die kindgerechte Grafik mit mindestens einer der Formen im Wesentlichen komplementär ist.

17. Einweg-Fausthandschuh aus Vliesstoff nach Anspruch 1, wobei der Gebrauchsindikator eine kindgerechte Grafik ist.

18. Einweg-Fausthandschuh aus Vliesstoff nach einem der vorgenannten Ansprüche, wobei der Einweg-Fausthandschuh aus Vliesstoff Bestandteil eines Systems von Verbraucherprodukten ist.

## Revendications

1. Moufle nontissée jetable prévue pour s'ajuster à la main d'un enfant, ladite moufle comprenant :
(a) un premier et un deuxième éléments de feuille non-tissés dans un rapport de superposition de couches, lesdits éléments définissant un volume intérieur pour recevoir ladite main de l'enfant, chacun desdits premier et deuxième éléments de feuille non-tissés incluant une surface externe, ayant une surface intérieure opposée, un bord supérieur, un bord inférieur opposé audit bord supérieur, et un premier et un deuxième bords latéraux opposés, lesdits premier et deuxième éléments de feuille non-tissés fixés en permanence l'un à l'autre le long de la périphérie dudit bord supérieur et des deux premier et deuxième bords latéraux opposés, lesdits bords inférieurs étant non fixés de façon à fournir un accès substantiel s'ouvrant vers ledit volume intérieur pour insérer aisément ladite main de l'enfant dedans ;
(b) au moins un motif pour enfant disposé sur au moins un desdits premier et deuxième éléments de feuille, ladite moufle comprenant de préférence au moins deux motifs pour enfant ; **caractérisé en ce que** ladite moufle comprend en outre une composition bénéfique, dans laquelle au moins un desdits premier et deuxième éléments de feuille renferme de façon libérable ladite composition bénéfique, ladite composition bénéfique étant de préférence choisie dans le groupe constitué de compositions de nettoyage, de compositions hydratantes, de compositions médicinales, de compositions de produit de beauté, de compositions pour la toilette, de compositions germicides, de compositions parfumées, et leurs combinaisons ;
(c) un indicateur d'usage, ledit indicateur d'usage fournissant de préférence un signal visuel pendant l'usage dudit article indiquant qu'au moins une partie de ladite composition bénéfique a été libérée dudit élément nontissé, ledit indicateur d'usage étant plus préférablement choisi dans le groupe constitué d'un indicateur de changement de pH, un indicateur de changement de température, un indicateur de changement de temps et leurs combinaisons.

2. Moufle nontissée jetable selon la revendication 1, dans laquelle ledit motif pour enfant est disposé sur ledit premier élément de feuille nontissé.

3. Moufle nontissée jetable selon la revendication 1, dans laquelle ledit motif pour enfant est disposé sur ledit deuxième élément de feuille nontissé.

4. Moufle nontissée jetable selon l'une quelconque des revendications précédentes, dans laquelle ledit motif pour enfant est sous la forme de photos, dessins, caricatures, symboles et leurs combinaisons.

5. Moufle nontissée jetable selon la revendication 1, où ladite moufle nontissée jetable est sans pouce.

6. Moufle nontissée jetable selon l'une quelconque des revendications 1 à 4, dans laquelle ledit volume intérieur est divisé en au moins deux parties où au moins une desdites parties est adaptée pour entourer le pouce d'un enfant.

7. Moufle nontissée jetable selon l'une quelconque des revendications précédentes, dans laquelle ledit motif pour enfant est attractif pour un enfant d'environ trois ans à environ cinq ans.

8. Moufle nontissée jetable selon l'une quelconque des revendications 1 à 6, dans laquelle ledit motif pour enfant est attractif pour un enfant d'environ six ans à environ neuf ans.

9. Moufle nontissée jetable selon l'une quelconque des revendications précédentes, dans laquelle ledit motif pour enfant est choisi dans le groupe constitué de motifs attractifs pour les filles, de motifs attractifs pour les garçons, de motifs unisexes et de motifs attractifs pour les deux sexes.

10. Moufle nontissée jetable selon l'une quelconque des revendications précédentes, dans laquelle ledit motif pour enfant comprend un motif de personnage, ledit motif de personnage ayant de préférence une forme choisie dans le groupe constitué de photos, dessins, caricatures, symboles et leurs combinaisons, ledit motif de personnage étant plus préférablement sous la forme d'une caricature choisie dans le groupe constitué de caricatures d'animaux, de caricatures d'objets et leurs combinaisons, et le plus préférablement ledit motif de personnage est une caricature d'un animal, dans laquelle l'animal est choisi dans le groupe constitué d'amphibiens, de tortues, de singes, d'automobiles et leurs combinaisons.

11. Moufle nontissée jetable selon l'une quelconque des revendications précédentes, dans laquelle ledit motif pour enfant est un motif déposé.

12. Moufle nontissée jetable selon l'une quelconque des revendications précédentes, dans laquelle au moins une partie d'au moins un desdits premier et deuxième éléments de feuille non-tissés comprend au moins une région soulevée, ladite région soulevée ayant de préférence une forme choisie dans le groupe constitué de logos, inscriptions, marques, motifs géométriques, images et leurs combinaisons.

13. Moufle nontissée jetable selon les revendications 1 à 11, dans laquelle au moins une partie d'au moins un desdits premier et deuxième éléments de feuille non-tissés comprend au moins une région abaissée, ladite région abaissée ayant de préférence une forme choisie dans le groupe constitué de logos, inscriptions, marques, motifs géométriques, images et leurs combinaisons.

14. Moufle nontissée jetable selon la revendication 12 ou 13, dans laquelle ladite forme est disposée de manière aléatoire sur au moins un desdits premier et deuxième éléments de feuille.

15. Moufle nontissée jetable selon la revendication 12 ou 13, dans laquelle ladite forme est disposée dans un motif répétitif.

16. Moufle nontissé jetable selon la revendication 12 ou 13, dans laquelle ledit motif pour enfant est essentiellement complémentaire avec au moins une desdites formes.

17. Moufle nontissée jetable selon la revendication 1, dans laquelle ledit indicateur d'usage est un motif pour enfant.

18. Moufle nontissée jetable selon l'une quelconque des revendications précédentes, où ladite moufle nontissée jetable est un composant d'un système de produits de consommation.
